# EUROPÄISCHE PATENTSCHRIFT

(11) **EP 2 427 470 B1**
(45) Veröffentlichungstag und Bekanntmachung des Hinweises auf die Patenterteilung: **20.08.2014**
(21) Anmeldenummer: 10726414.5
(22) Anmeldetag: 06.05.2010
(51) Int. Cl.: C07H 19/073

(54) **VERFAHREN ZUR FESTPHASENGESTÜTZTEN HERSTELLUNG PHOSPHATVERBRÜCKTER NUCLEOSID-KONJUGATE**
METHOD FOR THE SOLID PHASE-BASED PRODUCTION OF PHOSPHATE-BRIDGED NUCLEOSIDE CONJUGATES
PROCÉDÉ DE PRÉPARATION BASÉE SUR UNE PHASE SOLIDE DE CONJUGUÉS DE NUCLÉOSIDES À PONT PHOSPHATE.

(30) Priorität: 07.05.2009 DE 102009020261
(43) Veröffentlichungstag der Anmeldung: 14.03.2012
(73) Patentinhaber: Universität Hamburg, 20146 Hamburg (DE)
(72) Erfinder: MEIER, Chris, 21635 Jork (DE); TONN, Viktoria, 22083 Hamburg (DE)
(74) Vertreter: Stüven, Ralf
(86) Internationale Anmeldenummer: PCT/DE2010/000508
(87) Internationale Veröffentlichungsnummer: WO 2010/127666

(56) Entgegenhaltungen:
- V. TONN, C. MAIE: "Solid Phase Synthesis of Nucleoside Diphosphate Glyocpyranoses and Phosphorylated Biomolecules" 15TH EUROPEAN CARBOHYDRATE SYMPOSIUM, PA 067, 19. Juli 2009 (2009-07-19), - 24. Juli 2009 (2009-07-24) Seite 249, XP002596654 Vienna, Austria
- WENDICKE S ET AL: "Effiziente Synthese von Nucleosiddiphosphat-Glycopyranosen" ANGEWANDTE CHEMIE,, Bd. 120, 1. Januar 2008 (2008-01-01), Seiten 15223-1525, XP002554866 in der Anmeldung erwähnt
- GAUR R K ET AL: "NOVEL SOLID PHASE SYNTHESIS OF 2'-O-METHYRIBONUCLEOSIDE 5'-TRIPHOSPHATES AND THEIR ALPHA-THIO ANALOGUES" TETRAHEDRON LETTERS, ELSEVIER, AMSTERDAM, NL LNKD- DOI:10.1016/S0040-4039(00)92072-0, Bd. 33, Nr. 23, 2. Juni 1992 (1992-06-02), Seiten 3301-3304, XP000601548 ISSN: 0040-4039 in der Anmeldung erwähnt
- PARANG K ET AL: "Selective diphosphorylation, dithiodiphosphorylation, triphosphorylation, and trithiotriphosphorylation of unprotected carbohydrates and nucleosides" ORGANIC LETTERS, AMERICAN CHEMICAL SOCIETY, US LNKD- DOI:10.1021/OL0521432, Bd. 7, 1. Januar 2005 (2005-01-01), Seiten 5589-5592, XP002464531 ISSN: 1523-7060 in der Anmeldung erwähnt
- Viktoria Caroline Tonn: "Festphasen-gebundene cycloSal-Nucleotide zur Synthese phosphorylierter Biomoleküle", 2011, Hamburg
- CÉLINE CRAUSTE ETAL.: "Insights into the soluble PEG-supported synthesis of cytosine-containing nucleoside 5'-mono-, di-, and triphosphates", JOURNAL OF ORGANIC CHEMISTRY, vol. 74, 2009, pages 9165-6172,

## Beschreibung

Die Erfindung betrifft ein Verfahren zur festphasengestützten Herstellung phosphatverbrückter Nucleosid-Konjugate, insbesondere von Nucleosiddiphosphat-Zuckern, (poly)phosphorylierten Nucleosiden, Dinucleosid-Polyphosphaten und Nucleotid-Zucker-Konjugaten.

Phosphatverbrückte Nucleosid-Konjugate sind in der Natur von großer Bedeutung. Sie sind nicht nur maßgeblich an stoffwechselenergetischen Vorgängen beteiligt, sondern bei nahezu allen Biosynthesen als Metaboliten vertreten. So spielen beispielsweise Dinucleosid-Polyphosphate bei diversen biologischen Funktionen eine essentielle Rolle, z.B. als Cofaktoren oder als Signal- und Regulatormoleküle. Nikotinamidadenindinucleotid (NAD) oder Flavinadenindinucleotid (FAD) sind zum Beispiel wichtige Coenzyme, die als Wasserstoffüberträger entscheidend an der Zellatmung beteiligt sind, und Verbindungen wie das im Folgenden wiedergegebene Diadenosintetraphosphat (Ap₄A) 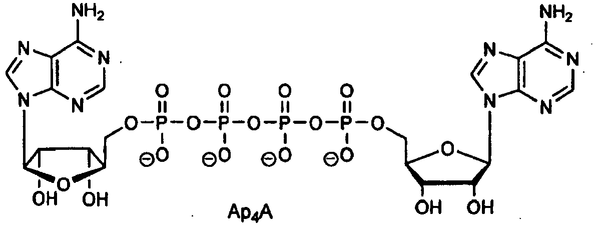 sowie verwandte Verbindungen besitzen großes diabetisches Potential. Daher ist beispielsweise die Entwicklung und Herstellung chemisch stabiler Analoga mit Blick auf die Bedeutung der Gluconeogenese, der Glucoseaufnahme, des Lipidstoffwechsels und der Blutdruckregulation von großem Interesse.

Auch so genannte Nucleosiddiphosphat- oder XDP-Zucker, eine besondere Klasse von Kohlenhydrat-Derivaten, sind von erheblicher Bedeutung. Eine allgemeine Formel für solche Zucker ist im Folgenden wiedergegeben:

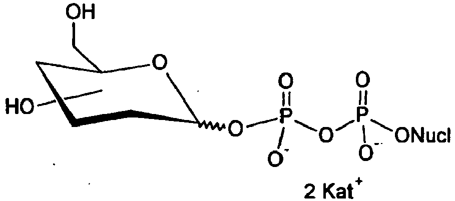

Nucl steht dabei für Nucleosid, Kat⁺ für ein Kation. Diese allgemein auch als Zuckernucleotide bezeichneten Verbindungen bestehen aus einem Nucleosid, das über eine Pyrophosphat-Einheit mit einem Pyranose-Derivat über das anomere Zentrum verbrückt ist. Die anomeren Phosphatester-Gruppen der Nucleosiddiphosphatpyranosen aktivieren den Glykosylrest für enzymatische Übertragungsreaktionen, während der Nucleosid-Rest Glykosyl-Transferasen oder Isomerasen als zusätzliches Erkennungsmerkmal für spezifische Umsetzungen dient. Diese aktivierten Monosaccharide erfüllen zwei, für den aufbauenden Stoffwechsel essentielle Funktionen. So sind sie an der Biosynthese von Desoxyzuckern, von Aminodesoxyzuckern und verzweigtkettigen Zuckern beteiligt. Darüber hinaus stellen XDP-Zucker die entscheidenden Bausteine zur Biosynthese von Oligosacchariden sowie des größten Teils der Polysaccharide dar.

Als weiteres Beispiel sind die natürlich vorkommenden Ribo- und Desoxyribonucleosidtriphosphate (NTPs und dNTPs) zu erwähnen. Sie stellen die elementaren Bausteine für die enzymkatalysierte RNA- und DNA-Synthese in vivo und in vitro dar, während ihren Analoga ein enormes Potential als Inhibitoren bei vielen biologischen Prozessen (z.B. Prozessen, an denen DNA-Polymerasen beteiligt sind) oder als Chemotherapeutika zukommt. Aus diesem Grund besteht ein besonders großes Interesse an einem synthetischen Zugang zu diesen Verbindungen. Allerdings ist nicht nur die Synthese von Nucleosidtriphosphaten, sondern inbesondere auch deren Isolierung ein großes Problem. Durch die Umsetzung von hochgeladenen Reagenzien, wie z.B. Pyrophosphat, mit lipophilen geschützten Nukleosid-Derivaten wird zum einen die Darstellung dieser Verbindungen erheblich erschwert, zum anderen ist die Isolierung eines geladenen, wasserlöslichen Produktes aus einer Mischung von hydrophilen und hydrophoben Bestandteilen sehr schwierig. Zudem sind Nukleosidtriphosphate aufgrund ihrer energiereichen Anhydridbindungen hydrolyseempfindlich. Ihre Stabilität hängt sowohl vom Gegenion als auch vom pH-Wert des Mediums ab (Z. Milewska, H. Panusz, Anal. Biochem. 1974, 57,8-13).

Im Stand der Technik findet sich eine Reihe von Verfahren zur Darstellung dieser Verbindungsklasse. Die am meisten genutzte Synthesestrategie zur chemischen Darstellung von Nucleosidtriphosphaten basiert auf dem nucleophilen Angriff eines Pyrophosphat-Salzes auf ein aktiviertes Nucleosidmonophosphat (M. Yoshikawa, T. Kato, T. Takenishi; Tetrahedron Lett 1967, 50, 5065-68). Pyrophosphatsalze wie beispielsweise tris-(tetra-n-Butylammonium)-hydrogenpyrophosphat sind kommerziell erhältlich, die aktivierten Nucleotide müssen jedoch stets synthetisiert werden.

Die auf M. Yoshikawa et al (s.o.). zurückgehenden Nucleosidphosphorchloridate wurden von Ludwig (J. Ludwig; Acta. Biochim. et Biophys. Acad. Sci. Hung. 1981, 16, 131-133.) und anderen (L. Rutil; Y.C. Cheng, Mol. Pharmacol. 1981, 20, 415-422) direkt mit bis(tri-n-Butylammonium)pyrophosphat umgesetzt. Der nucleophile Angriff des Pyrophosphat-Ions führt zur Bildung des cyclischen Trimetaphosphatalkyl-Intermediats (W. Feldmann; Chem. Ber. 1966, 99(10), 3251-3259; A. W. Schwartz; J. Chem. Soc., Chem. Commun. 1969, 1393. Ludwig; Bioact. Mol. 1987,3,201-204), das im nachfolgenden Hydrolyseschritt das Nucleosidtriphosphat liefert.

Allerdings beschränkt sich diese Methode auf Nucleosid-Derivate, die unempfindlich gegenüber den Bedingungen der Monophosphorylierung nach M. Yoshikawa sind, was den Einsatz besonders von modifizierten Purin-Nucleosiden nur sehr eingeschränkt möglich macht (W. Wu, D.E. Bergstrom, V.J. Davisson, J. Org. Chem. 2003,68,3860-3865). Auch Alken-funktionalisierte Nucleoside können auf diesem Wege beispielsweise nicht phosphoryliert werden, da das bei der Reaktion aus P(O)Cl₃ entstehende HCl an die Alkenfunktion addiert (T. Koväcs, L. Ötvös.; Tetrahedron Lett. 1988,29,4525-4528). Zudem ergeben sich weitere Probleme aus der mangelnden Selektivität von P(O)Cl₃ als Phosphorylierungsreagenz (W.H. Dawson, RL. Cargill, R.B. Dunlap, J. Carbohydr. Nucleosides Nucleotides 1977,4, 363-375)

Weitere im Stand der Technik intensiv genutzte Synthesewege für Nucleosidtriphosphate verwenden Nucleosidphosphormorpholidate (S. Roseman, J.J. Distler, J.G. Moffatt, H.G. Khorana; J. Am. Chem. Soc. 1961,83,659-663; J.G. Moffatt, H.G. Khorana; J. Am. Chem. Soc. 1954, 80, 3756-3761.), -amidate (J. Tomasz, A. Simoncsits, M. Kajtar. R.M. Krug, A. Shatkin, J. Nucl. Acids Res. 1978, 5, 2945-2957; A. Simoncsits, J. Tomasz, J. Nucl. Acids Res. 1975, 2, 1223-1233) oder imidazolidate (D.E. Hoard, D.G. Ott, J Am. Soc. Chem. 1965, 87, 1785-1788; M. Shimazu, K. Shinozuka, H. Sawai, Tetrahedron Lett. 1990,31,235-238) als aktivierte Nucleotide. Die Umsetzung zum Triphosphat braucht jedoch oft einige Tage und die chemischen Ausbeuten sind in vielen Fällen eher moderat. Bei diesen Methoden verdrängt bis(tri-n-Butylammonium)pyrophosphat ebenfalls in einer nucleophilen Substitution den Morpholin-, Amin- beziehungsweise Imidazol-Rest aus dem 5'-Nucleosylphosphat-Derivat.

Ein weiterer Weg zur Synthese von Nucleosid-5'-triphosphaten basiert auf dem Einsatz von aktivierten Nucleosid-5'-phosphiten oder -phosphoramiditen. Frühzeitig wurden dazu Arbeiten von J. Ludwig und F. Eckstein veröffentlicht (J. Ludwig, F. Eckstein, J. Org. Chem. 1989, 54, 631-635), bei denen Nucleoside mit einem Salicylssäurephosphorchloridit zu reaktiven Nucleosid-5'-phosphiten umgesetzt wurden, die anschließend in situ mit bis(tri-n-Butylammonium)-pyrophosphat zunächst zu einem cyclischen Intermediat reagieren. Die nachfolgende Oxidation/Hydrolyse liefert dann das entsprechende Triphosphat. Ein Vorteil dieser Syntheseroute besteht in der höheren Reaktivität von P(III)-Reagenzien. Von Nachteil ist allerdings der nötige Hydrolyse/Oxidationsschritt ausgehend vom Intermediat.

Ein Weg zur Synthese von Nucleosiddiphosphat(NDP)-Zuckern unter Verwendung von so genannten cyclo-Saligenyl(cycloSal)-Nucleosidphosphattriestem ist von Wendicke et al. (Angew. Chem. 2008, 120, 1523-1525) beschrieben worden. Bei diesen cycloSal-Nucleosidphosphattriestern, die auch als cycloSal-NMPs, cycloSal-Nucleotide oder cycloSal-Triester bezeichnet werden, handelt es sich um cyclische Phosphattriester-Derivate, bei denen ein Salicylalkohol mit einem Nucleosidmonophosphat zweifach cyclisch verestert ist. Die Grundstruktur der von Wendicke et al. verwendeten cyclo-Saligenyl-Nucleotide ist im Folgenden dargestellt.

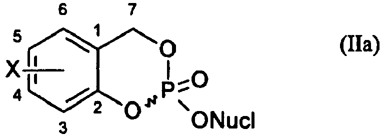

Bei dem Verfahren wurde der cycloSal-Triester 5-Nitro-cycloSal-3'-O-acetyl-thymidin-monophosphat mit entsprechenden Glycopyranosyl-1-phosphaten in wasserfreiem DMF in einem Molverhältnis von 1:1,2 gemischt. Die resultierenden NDP-Zucker wurden nach 3-5 h bei Raumtemperatur in Ausbeuten von 40-60% erhalten.

Ein festphasengestütztes Verfahren zur Synthese von Nucleosiddi- und -triphosphaten ist von Ahmedibeni und Parang (Y. Ahmadibeni; K. Parang; Selective Diphosphorylation, Dithiodiphosphorylation, Triphosphorylation, and Trithiotriphosphorylation of Unprotected Carbohydrates and Nucleosides; Org. Lett. 2005, 7(25), 5589-5592) beschrieben worden. Die Synthese beruht auf Di- und Triphosphitylierungsreagenzien, welche an die Festphase Aminomethylpolystyrol gebunden sind. Die Di- und Triphosphitylierungsreagenzien müssen zunächst in dreistufigen Synthesen hergestellt werden. Nach Reaktion mit dem gewünschten Nucleosid werden die Phosphit- zu Phosphateinheiten oxidiert, d.h. die zu phosphorylierenden Nucleoside oder Zucker müssen unter den harschen Oxidationsbedingungen (tBuOOH) sowie unter der darauffolgenden Abspaltung der Cyanogruppen stabil sein. Nach Abspaltung von der Festphase wurden Rohprodukte mit schwankender Reinheit sowie moderaten Ausbeuten erzielt. Die schwankenden Ausbeuten zeigen, dass die Methode nicht generell anwendbar ist, um hohe Ausbeuten zu erzielen.

Von Ahmadibeni und Parang wurde darüber hinaus eine Methode publiziert, die Nucleosid-5'-O-B-triphosphate liefert (Y. Ahmadibeni; K. Parang; Application of a Solid-Phase β-Triphosphitylating Reagent in the Synthesis of Nucleoside ß-Triphosphates; J. Org. Chem. 2006,71,5837 - 5839). Auch hier ergaben sich aber schwankende Reinheiten und moderate Ausbeuten. Zudem ist die Methode auf eine ß-Phosphorylierung beschränkt.

Von Gaur et al. wurde eine Methode veröffentlicht, bei der das Nucleosid an die Festphase gebunden und zum Di- bzw. Triphosphat phosphoryliert wird (R. K. Gaur, B. S. Sproat; G. Krupp; Novel Solid Phase Synthesis of 2'-O-Methylribonucleoside 5'-triphosphates and their α-thio analogues; Tetrahedron Lett. 1992, 33(23), 3301-3304). Die Methode erfordert nach Abspaltung des Produktes von der Festphase eine chromatographische Reinigung an Sephadex und liefert nur moderate Ausbeuten des Nucleosid-5'-triphosphats von 60-65%. Ebensolche Ausbeuten sollen auch mit der Methode von Burgess und Cook erzielt werden können (K. Burgess; D. Cook; Syntheses of Nucleoside Triphosphates; Chem. Rev. 2000,100,2047-2059). Beide zuletzt genannten Methoden beinhalten die Verwendung des Harzes CPG ("controlled pore glas") als Festphase, welches im Verhältnis zu Festphasen auf Polystyrolbasis teuer ist.

Aufgabe der vorliegenden Erfindung ist es, ein Verfahren zur Herstellung von phosphatverbrückten Nucleotid-Biokonjugaten, insbesondere von Nucleosiddiphosphat-Zuckern, (poly)phosphorylierten Nucleosiden, Dinucleosid-Polyphosphaten und Nucleosid-Zucker-Konjugaten, bereit zu stellen, das in einem weiten Bereich anwendbar ist und die Verbindungen in möglichst einfacher und schneller Weise in möglichst hoher Ausbeute und Reinheit liefert. Darüber hinaus ist es Aufgabe der Erfindung, Verbindungen bereitzustellen, die in vorteilhafter Weise zur Herstellung entsprechender phosphatverbrückten Nucleotid-Biokonjugate eingesetzt werden können.

Gelöst wird die Aufgabe durch das in Anspruch 1 angegebene Verfahren und die in Anspruch 11 angegebene Verbindung. Bevorzugte Ausführungsformen sind jeweils in den Unteransprüchen angegeben.

Unter "phosphatverbrückten Nucleosid-Konjugaten" werden hier Verbindungen der allgemeinen Formel (I) 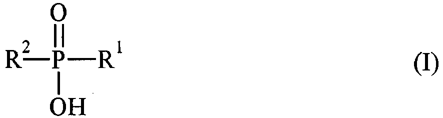 oder Salze davon verstanden, wobei R¹ Nucl ist und wobei Nucl ein Nucleosid oder Nucleosidanalogon ist.

R² ist eine beliebige organische Verbindung oder Phosphat oder Pyrophosphat. Bevorzugt ist R² eine in einer lebenden Zelle vorkommende oder eine dazu analoge Verbindung bzw. ein entsprechender Verbindungsrest, beispielsweise ein Alkohol, ein Zucker, ein Lipid, ein Nucleosid, ein Nucleosidmono-, -di- oder -triphosphat, Phosphat oder Pyrophosphat bzw. ein Alkohol-, Zucker-, ein Lipid-, Nucleosid-, Nucleosidmono-, -di- oder -triphosphat-, Phosphat- oder Pyrophosphatrest. In diesem Fall wird auch von Biokonjugaten gesprochen.

Es ist überraschend gefunden worden, dass phosphatverbrückte Nucleosid-Konjugate auf einfache Weise in sehr hohen Ausbeuten und Reinheiten erhältlich sind, wenn das oben skizzierte "cycloSal-Konzept" in erfinderischer Weise modifiziert wird. Bei dem Verfahren gemäß der vorliegenden Erfindung wird eine Verbindung der obigen allgemeinen Formel (I), oder ein Salz davon, vorzugsweise ein pharmazeutisch annehmbares Salz davon, hergestellt, indem an eine Verbindung der allgemeinen Formel (II) 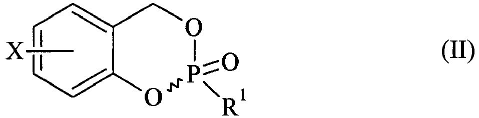 ein Linker (L) addiert und eine Verbindung der folgenden Formel (III) 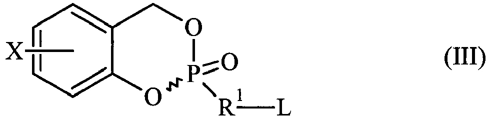 hergestellt und die Verbindung (III) durch kovalente Bindung des Linkers (L) an eine mindestens eine Aminogruppe -NH₂ enthaltende Festphase immobilisiert wird, die immobilisierte Verbindung (III) mit einem mit R² identischen oder R² umfassenden Nucleophil umgesetzt und der Linker (L) vom Rest R¹ abgespalten wird.

In einer alternativen Ausgestaltung des erfindungsgemäßen Verfahrens liegt der Linker (L) bereits an eine Festphase gekoppelt vor. Bei dieser Ausgestaltung wird daher der kovalent an eine Festphase gebundene Linker (L) an die Verbindung der allgemeinen Formel (II) addiert, und das entsprechende Reaktionsprodukt, d.h. die über den Linker (L) an die Festphase gekoppelte Verbindung (II), wird dann mit einem mit R² identischen oder R² umfassenden Nucleophil umgesetzt.

R¹ und R² sind dabei wie oben definiert. X kann ein beliebiger Elektronenakzeptor, H oder OMe sein und ist vorzugsweise H, OMe, MeSO₂, Keton, C=O, COOH, Formyl, Ester, NO₂ oder Halogen, wobei Me für Methyl steht. Für den Fall, dass eine Carbonylgruppe C=O im Rest X vorhanden ist, ist es bevorzugt, dass diese unmittelbar am aromatischen Ring sitzt. Der aromatische Ring in Verbindung (II) kann ein oder mehrfach mit X substituiert sein, wobei die Substituenten X bei Mehrfachsubstitution gleich oder verschieden sein können. Die Verbindung gemäß Formel (II) kann auch am C-Atom 7 (zur Nummerierung s. Formel IIa) substituiert sein, beispielsweise durch Methyl, i-Propyl, tert-Butyl oder andere Alkylsubstituenten. Auch der aromatische Ring kann gegebenenfalls noch weitere Substituenten als X, beispielsweise Alkyl- oder Arylsubstituenten, aufweisen.

Unter einem "Linker" (L) wird hier eine organische Verbindung verstanden, über die die Verbindung (II) und später die Verbindung (I) kovalent an die Festphase gebunden wird. Der Linker weist vorzugsweise mindestens zwei funktionelle Gruppen, z.B. Carboxylgruppen -COOH, auf und fungiert zunächst als Verbindungsstück oder Abstandhalter zwischen der Verbindung (II) und der Festphase und ist sowohl mit der Verbindung (II) als auch mit der Festphase kovalent verbunden. Nach Umsetzung mit einem geeigneten Nucleophil werden mit Hilfe des Linkers phosphatverbrückte Nucleosid-Konjugate gemäß der vorliegenden Erfindung an die Festphase gekoppelt.

Ein bei dem vorliegenden Verfahren bevorzugt verwendeter Linker ist beispielsweise ein Linker gemäß der allgemeinen Formel (IV):

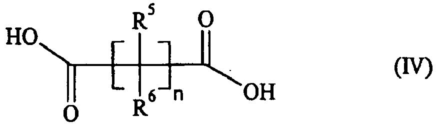

R⁵ und R⁶ sind dabei, jeweils unabhängig voneinander, H, substituierte oder unsubstituierte Alkyl- oder Arylreste, und n ist 1, 2, 3, 4, 5, 6, 7, 8, 9 oder 10. "Unabhängig voneinander" in Bezug auf R⁵ und R⁶ soll auch bedeuten, dass bei n>1 mehrere Reste R⁵ und mehrere Reste R⁶ jeweils unabhängig voneinander H, substituierte oder unsubstituierte Alkyl- oder Arylreste sein können, so dass ein erster Rest R⁵⁽¹⁾ beispielsweise H, ein weiterer Rest R⁵⁽²⁾ z.B. Methyl sein kann. Die kovalente Bindung erfolgt dabei über die beiden Carboxylgruppen.

Bei dem erfindungsgemäßen Verfahren können aber auch andere Linker eingesetzt werden, beispielsweise Benzoyl-Linker, z.B. der Struktur -OC-C₆H₄-O- oder -OC-C₆H₄-. Solche Benzoyl-Linker können beispielsweise in Form des jeweiligen Carbonsäurehalogenids (z.B. Carbonsäurechlorids) mit der Verbindung (II) in Reaktion gebracht werden. Dem Fachmann sind Linker grundsätzlich bekannt und er wird, gegebenenfalls nach entsprechenden Routineversuchen, einen für den jeweiligen Zweck geeigneten Linker auswählen.

Für den Fachmann ist in diesem Zusammenhang selbstverständlich, dass die OH-Gruppen der Carboxylgruppen des Linkers (IV) bei der Addition an die Verbindung (III) und Bindung an die Festphase abgespalten werden und der Begriff Linker daher auch den entsprechenden Rest, beispielsweise gemäß der allgemeinen Formel (IVa) 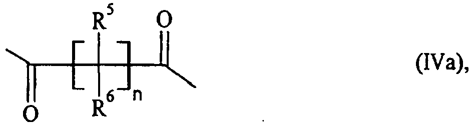 umfasst. Entsprechendes gilt selbstverständlich im Falle der Benzoyl-Linker. Auch hier umfasst der Begriff sowohl den gebundenen Rest als auch die "freie" bzw. nur an die Festphase gekoppelte Verbindung".

Wenn hier der Begriff "Succinyl-Linker" verwendet wird, wird darunter eine Verbindung der allgemeinen Formel (IVb) 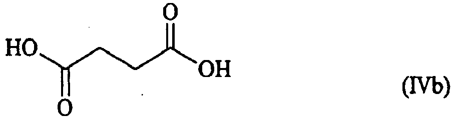 oder ein entsprechender Rest (s. oben) verstanden. Ein Succinyl-Linker kann beispielsweise an die Verbindung (II) addiert werden, indem Bernsteinsäureanhydrid 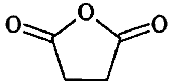 mit der Verbindung (II) in Reaktion gebracht wird.

Ein großer Vorteil des erfindungsgemäßen Verfahrens ist dessen breite Anwendungsmöglichkeit auf eine Vielzahl von Verbindungen. Mit dem erfindungsgemäßen Verfahren können beliebige phosphatverbrückte Nucleosid-Konjugate in hoher Ausbeute und Reinheit hergestellt werden, beispielsweise Nucleosiddiphosphat-Glycopyranosen. Mit dem Einsatz von Phosphat- und Pyrophosphatsalzen können beispielsweise Nucleosiddi- und Nucleosidtriphosphate hergestellt werden. Mit dem erfindungsgemäßen Verfahren können in vorteilhafter Weise auch Nukleosidanaloga hergestellt werden, die beispielsweise als "Propharmaka" eingesetzt werden können. "Propharmaka" sind Wirkstoffvorläufer, welche den eigentlichen Wirkstoff erst später unter Abspaltung von Maskierungsgruppen freisetzen.

Darüber hinaus sind neben Dinucleosid-Monophosphaten auch Dinucleosid-Polyphosphate gemäß der allgemeinen Formel (V) 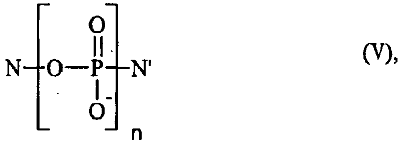 abgekürzt NpₙN', herstellbar. N und N' stehen hier für Nucleosid oder Nucleosidanalogon, wobei N und N' gleich oder verschieden sein können. P steht für Phosphat, der Index n gibt die Zahl der miteinander verknüpften Phosphatreste an und beträgt vorzugsweise 2, 3 oder 4. Ein Beispiel für ein Dinucleosid-Polyphosphat ist das oben bereits erwähnte Diadenosintetraphosphat (Ap₄A). Auch NAD oder FAD sind Dinucleosid-Polyphosphate im Sinne der vorliegenden Erfindung.

Zudem ist es mit Hilfe des erfindungsgemäßen Verfahrens auch möglich, Zucker-Nukleosid-Biokonjugate herzustellen.

"Organische Verbindungen" sind alle Verbindungen des Kohlenstoffs mit Kohlenstoff und mit anderen Elementen (mit Ausnahme von Kohlenstoffdioxid, Kohlenstoffmonoxid, Kohlensäure und ihren Carbonaten sowie Cyaniden, Isocyaniden, Cyanaten und Isocyanaten von Metallen). Beispiele für organische Verbindungen sind Kohlenwasserstoffe, d.h. Verbindungen von Kohlenstoff und Wasserstoff, Alkohole, Aldehyde, Ketone, Carbonsäuren, Amine, Amide, Nitroverbindungen, Nitrile, Alkanthiole, Sulfide, Sulfate, Phosphate, Phosphine, metallorganische Verbindungen, aliphatische Kohlenwasserstoffe, acyclische Kohlenwasserstoffe, gesättigte (Alkane), ungesättigte (Alkene und Alkine), cyclische Kohlenwasserstoffe, einfache oder kondensierte aromatische Kohlenwasserstoffe (Aromaten), Heterocyclen, biochemische Verbindungen (Aminosäuren, Proteine, Nucleoside, Nukleotide, Kohlenhydrate, Lipide) usw.

Unter einem "Carbocyclus" werden cyclische Verbindungen verstanden, deren ringbildende Atome ausschließlich aus C-Atomen bestehen.

Ein "Heterocyclus" ist eine cyclische Verbindung mit ringbildenden Atomen aus mindestens zwei verschiedenen chemischen Elementen. Insbesondere wird hierunter eine ringförmige organische Verbindung verstanden, in deren Ringgerüst mindestens ein Kohlenstoffatom durch ein anderes Element, d.h. ein Heteroatom, beispielsweise durch Stickstoff, Sauerstoff und/oder Schwefel ersetzt ist. Ein Ringgerüst kann aus ein oder mehreren miteinander verbundenen Ringen bestehen und ein oder mehrere gleiche oder verschiedene Heteroatome enthalten.

Der Begriff "Nucleophil" ist dem Fachmann bekannt und hat hier die dem Fachmann geläufige Bedeutung. Insbesondere wird hier unter einem Nucleophil ein Molekül verstanden, das einen negativ polarisierten Bereich, eine negativ polarisierte funktionelle Gruppe oder ein freies E-lektronenpaar, in der Regel in einem energiereichen Orbital, enthält. Dabei umfasst der Begriff auch Moleküle, die im Verhältnis zu einem betrachteten Reaktionspartner bzw. zu einem Bereich des Reaktionspartners nucleophil, d.h. verhältnismäßig elektronenreicher sind. Der Reaktionspartner wird auch als Elektrophil bezeichnet, weil er Elektronen vom Nucleophil übernimmt. Nucleophile können kovalente Bindungen ausbilden, indem sie einem Reaktionspartner Elektronen zur Verfügung stellen. Dabei stammen die für die Bindung benötigten Elektronen regelmäßig allein vom Nucleophil. Nucleophile können negativ geladen (Anionen) sein. Beispiele für typische nucleophile Reagenzien sind Carbanionen, Anionen, Lewis-Basen, Aromaten, Alkohole, Amine, z.B. Aminosäuren, und Verbindungen mit olefinischen Doppelbindungen.

Die Stärke der Nucleophilie hängt beispielsweise vom Reaktionspartner, der Basizität, dem Lösungsmittel und sterischen Faktoren ab. Dem Fachmann sind die Faktoren, die die Nucleophilie einer Verbindung beeinflussen, gut bekannt und er kann daher leicht deren nucleophile Eigenschaften bestimmen. Die Nucleophilie eines Moleküls wird dabei vorteilhaft auf das am stärksten nucleophile Atom bzw. die am stärksten nucleophile funktionelle Gruppe bezogen.

Im Falle des als Elektrophil eingesetzten cycloSal-Phosphattriesters gemäß der obigen allgemeinen Formel (II) kann die Elektrophilie des Phosphoratoms über den Substituenten X am cycloSal-Aromaten gesteuert werden (s. C. Meier, J. Renze, C. Ducho, J. Balzarini, Curr. Topics in Med. Chem. 2002,2, 1111-1121, deren Offenbarung hier durch Inbezugnahme vollständige aufgenommen ist). Durch Einführung von Donor-Substituenten am aromatischen Ring wird die Elektrophilie verringert, Akzeptor-Substituenten erhöhen demgegenüber die Reaktionsgeschwindigkeit der Initialreaktion, d.h. die cycloSal-Ringöffnung.

Ein "Elektronenakzeptor" ist eine Verbindung, ein Bereich einer Verbindung oder eine funktionelle Gruppe, die Elektronen zu sich heranzieht und damit in einer Verbindung eine Ladungsverschiebung, d.h. Polarisierung, herbeiführt. Beispiele für Elektronenakzeptorgruppen sind MeSO₂, COOH, Ketone bzw. die Ketogruppe, Formyl, Ester bzw. die Estergruppe, NO₂ und Halogen (z.B. F, Cl, Br, I). Me steht für Methyl. Als Elektronenakzeptor bevorzugte Ester sind solche, deren Estergruppe möglichst dicht, bevorzugt unmittelbar am aromatischen Ring sitzen. Als Elektronenakzeptor bevorzugte Ketone sind solche, deren Ketogruppe möglichst dicht, bevorzugt unmittelbar am aromatischen Ring sitzen.

Ester sind Verbindungen, die die Estergruppe R'-COO-R" enthalten, wobei R' und R" beliebige substituierte oder nicht-substituierte, verzweigt- oder geradkettige Kohlenwasserstoffreste, beispielsweise Alkylreste oder Arylreste, sein können.

Ketone sind Verbindungen, die die Ketogruppe R'-CO-R" enthalten, wobei R' und R" beliebige substituierte oder nicht-substituierte, verzweigt- oder geradkettige Kohlenwasserstoffreste, beispielsweise Alkylreste oder Arylreste, sein können.

Unter einem "Nucleosid" werden hier organische Moleküle verstanden, die aus einem Zuckerrest (Zuckerkomponente) und einer organischen Base (Basenkomponente), z.B. einer heterocyclischen organischen Base, insbesondere einer stickstoffhaltigen heterocyclischen organischen Base, bestehen, die über eine glykosidische Bindung verbunden sind. Der Zuckerrest ist häufig eine Pentose, z.B. Desoxyribose oder Ribose, kann aber auch ein anderer Zucker sein, z.B. ein C₃-, C₄- oder C₆-Zucker. Insbesondere wird unter einem Nucleosid daher eine Verbindung der allgemeinen Formel (VI) 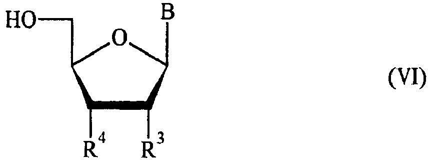 verstanden, wobei B eine stickstoffhaltige heterocyclische organische Base, z.B. eine Nucleobase, ist, und R³ und R⁴ unabhängig voneinander H oder OH sind.

Unter einer "Nucleobase" werden organische Basen verstanden, die in RNA oder DNA vorkommen. Bei den Nucleobasen handelt es sich häufig um Purine (R) und Pyrimidine (Y). Beispiele für Purine sind Guanin (G) und Adenin (A), Beispiele für Pyrimidine sind Cytosin (C), Thymin (T) und Uracil (U). Phosphorylierte Nucleoside, beispielsweise Nucleosidmonophosphate (NMP), Nucleosiddiphosphate (NDP) und Nucleosidtriphosphate (NTP), werden auch als Nucleotide bezeichnet. Die Phosphat-, Diphosphat- (Pyrophosphat-) bzw. Triphosphatgruppe ist in der Regel mit dem 5'-C-Atom der Zuckerkomponente des Nucleosids verknüpft, kann aber beispielsweise auch mit dem 3'-C-Atom verknüpft sein.

Unter einem "Nucleosidanalogon" wird hier eine Verbindung verstanden, die im menschlichen Körper natürlicherweise nicht vorktimmt, einem natürlicherweise im menschlichen Körper vorkommenden Nucleosid aber strukturell ähnlich ist, so dass es beispielsweise von der Zelle und/oder von viralen Enzymen im Wesentlichen entsprechend dem natürlichen Nucleosid verarbeitet, beispielsweise phosphoryliert und in einen RNA- oder DNA-Strang eingebaut werden kann. Ein Nucleosidanalogon kann selbst ein Nucleosid sein. Es kann aber beispielsweise auch eine andere Verbindung mit den obigen Eigenschaften sein, beispielsweise eine Verbindung aus einer heterocyclischen Base und einem acyclischen Rest und/oder einem Rest, der kein Zucker ist, oder eine Verbindung aus einer carbocyclischen Verbindung und einem Zuckerrest. Nucleosidanaloga sind entweder selbst Nucleoside im obigen Sinne oder Nucleosiden strukturell und/oder funktionell analog. Da Nucleosidanaloga nicht notwendig eine Zucker- bzw. Basenkomponente im engeren Sinne enthalten müssen, wird hier auch von einer zur Basenkomponente analogen Komponente (Basenanalogon) bzw. einer zur Zuckerkomponente analogen Komponente (Zuckeranalogon) gesprochen. Sofern hier von einer Zuckerkomponente oder Basenkomponente gesprochen wird, sollen die entsprechenden analogen Komponenten von Nucleosidanaloga mit erfasst sein, sofern sich aus dem Zusammenhang nicht eindeutig etwas anderes ergibt. Beispiele für Nucleosidanaloga sind beispielsweise AZT (3'-Azido-2',3'-didesoxythimidin, Azidothymidin), 2',3'-Didesoxyinosin (Didanosin), 2',3'-Didesoxycytidin (Zalticabin) und 2-Amino-9-((2-hydroxyethoxy)methyl)-1H-purin-6(9H)-on (Acyclovir). Auch Nucleosidphosphonate können Nucleosidanaloga sein.

Unter dem Begriff "Glycosylphosphat" wird ein phosphorylierter Glycosylrest verstanden. Ein "Glycosyl" ist eine Verbindung mit einer funktionellen Gruppe, die von einem Zucker durch Entfernung der Hemiacetal-Hydroxygruppe abgeleitet wurde. Beispiele für Glycosyl-1-phosphate sind: Glucose-1-Phosphat, Mannose-1-Phosphat, Galactose-1-Phosphat, 2-N-Acetyl-Glucosamin-1-Phosphat, 6-Desoxygulose-1-Phosphat, 2-N-Acetyl-Galactosamin-1-Phosphat, D-Fucose-1-Phosphat und L-Fucose-1-Phosphat; jeweils in der α- bzw. β-Konfiguration am anomeren Zentrum (bei Mannose gibt es nur die α-Form).

Der Begriff "Alkyl" beinhaltet gesättigte aliphatische Gruppen, einschließlich geradkettiger Alkylgruppen (z.B. Methyl, Ethyl, Propyl, Butyl, Pentyl, Hexyl, Heptyl und Octyl), verzweigtkettiger Alkylgruppen (z.B. Isopropyl, tert-Butyl, Isobutyl), Cycloalkyl- (z.B. alicyclische) Gruppen (z.B. Cyclopropyl, Cyclopentyl, Cyclohexyl, Cycloheptyl, Cyclooctyl), alkylsubstituierte Cycloalkylgruppen und cycloalkylsubstituierter Alkylgruppen. "Alkyl" beinhaltet ferner Alkylgruppen, die Sauerstoff-, Stickstoff-, Schwefel- oder Phosphoratome aufweisen, die ein oder mehrere Kohlenstoffatome des Kohlenwasserstoffgerüsts ersetzen. Der Begriff umfasst auch O-, N-, S- oder P-Alkylgruppen (z.B. -O-Methyl), d.h. Alkylgruppen, die über ein Sauerstoff-, Stickstoff-, Schwefel- oder Phosphoratom an eine Verbindung gebunden sind. Der Begriff "Alkyl" beinhaltet ebenfalls sowohl unsubstituierte Alkyle als auch substituierte Alkyle, wobei sich das letztere auf Alkylreste bezieht, die Substituenten aufweisen, die ein Wasserstoffatom an einem oder mehreren Kohlenstoffatomen des Kohlenwasserstoffgerüsts ersetzen. Solche Substituenten können zum Beispiel beinhalten: Alkyl, Alkenyl, Alkynyl, Halogen, Hydroxyl, Alkylcarbonyloxy, Arylcarbonyloxy, Alkoxycarbonyloxy, Aryloxycarbonyloxy, Carboxylat, Alkylcarbonyl, Arylcarbonyl, Alkoxycarbonyl, Aminocarbonyl, Alkylaminocarbonyl, Dialkylaminocarbonyl, Alkylthiocarbonyl, Alkoxyl, Phosphat, Phosphonato, Phosphinato, Cyano, Amino (einschließlich Alkylamino, Dialkylamino, Arylamino, Diarylamino und Alkylarylamino), Acylamino (einschließlich Alkylcarbonylamino, Arylcarbonylamino, Carbamoyl und Ureido), Amidino, Imino, Sulfhydryl, Alkylthio, Arylthio, Thiocarboxylat, Sulfate, Alkylsulfinyl, Sulfonato, Sulfamoyl, Sulfonamido, Nitro, Trifluoromethyl, Cyano, Azido, Heterocyclyl, Alkylaryl oder ein aromatischer oder heteroaromatischer Rest. Cycloalkyle können weiter substituiert sein, z.B. mit den oben angegebenen Substituenten. Ein "Alkylaryl"- oder ein "Aralkyl"-Rest ist ein Alkyl, das mit einem Aryl substituiert ist (z.B. Phenylmethyl (Benzyl)). "Alkyl" beinhaltet auch die Seitenketten von natürlichen und unnatürlichen Aminosäuren.

Unter "Aryl" werden Gruppen mit Aromatizität verstanden, einschließlich 5- und 6-gliedrigen aromatischen Einzelringgruppen, die null bis vier Heteroatome beinhalten können, sowie multizyklischen Systemen mit mindestens einem aromatischen Ring. Beispiele für Aryl-Gruppen beinhalten Benzol, Phenyl, Pyrrol, Furan, Thiophen, Thiazol, Isothiazol, Imidazol, Triazol, Tetrazol, Pyrazol, Oxazol, Isooxazol, Pyridin, Pyrazin, Pyridazin und Pyrimidin, und dergleichen. Darüber hinaus beinhaltet der Begriff "Aryl" multizyklische Aryl-Gruppen, z.B. trizyklische, bizyklische, z.B. Naphthalen, Benzoxazol, Benzodioxazol, Benzothiazol, Benzoimidazol, Benzothiophen, Methylenedioxyphenyl, Quinolin, Isoquinolin, Napthridin, Indol, Benzofuran, Purin, Benzofuran, Deazapurin oder Indolizin. Unter "Aryl" werden auch Aryl-Gruppen verstanden, die Heteroatome in der Ringstruktur aufweisen ("Heteroaryle"). Der aromatische Ring kann an ein oder mehreren Ringpositionen substituiert sein. Aryl-Gruppen können auch mit alizyklischen oder heterocyclischen Ringen, die nicht aromatisch sind, fusioniert oder verbrückt sein, so dass ein multizyklisches System gebildet wird (z.B. Tetralin, Methylenedioxyphenyl). Der Begriff umfasst auch O-, N-, S- oder P-Arylgruppen, d.h. Arylgruppen, die über ein Sauerstoff-, Stickstoff-, Schwefel- oder Phosphoratom an eine Verbindung gebunden sind.

Unter einem "Amin" werden hier Verbindungen des Typs R-NH₂, NH-R₂, N-R₃ und N-R₄⁺ verstanden, wobei R für einen substituierten oder unsubstituierten Alkyl- oder Arylrest steht, wobei im Falle mehrerer Reste diese verschieden oder gleich sein können. Die Reste können zu einem Ring geschlossen sein, so dass der Begriff auch cyclische Amine umfasst.

Unter dem hier verwendeten Begriff "Schutzgruppe" wird ein Molekül bzw. Molekülrest verstanden, das bzw. der eine funktionelle Gruppe in einer Verbindung während einer Umsetzung an anderer Stelle in der Verbindung blockiert und unerwünschte (Neben-)Reaktionen verhindert. Eine Schutzgruppe lässt sich idealerweise unter möglichst schonenden Bedingungen einführten, ist unter den Bedingungen der nachfolgenden Reaktion stabil und nach erfolgter Reaktion schonend wieder abspaltbar. Schutzgruppen sind dem Fachmann gut bekannt, so dass er bei Bedarf eine geeignete Schutzgruppe, gegebenenfalls nach Durchführung von Routineversuchen, leicht auffinden wird. Beispiele für eine Schutzgruppe sind die Methyl-, Acetyl- und Benzoylgruppe. Beispielsweise kann eine OH-Gruppe durch O-Methylierung oder O-Acetylierung vor einer Umsetzung geschützt werden.

In einer bevorzugten Ausführungsform des erfindungsgemäßen Verfahrens wird in Schritt a) ein Linker (L) gemäß der allgemeinen Formel (IV) 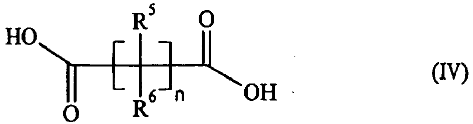 verwendet und eine Verbindung der allgemeinen Formel (IIIa) 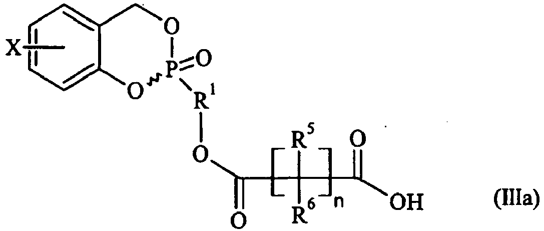 hergestellt, wobei R⁵ und R⁶, jeweils unabhängig voneinander, H, substituierte oder unsubstituierte Alkyl- oder Arylreste sind, und n = 1, 2, 3, 4, 5, 6, 7, 8, 9 oder 10 ist.

Bei dem Linker (IV) ist n vorzugsweise 2, und R⁵ und R⁶ sind jeweils beide H, so dass der Linker die Struktur HOOC-(CH₂)₂-COOH bzw. -OC-(CH₂)₂-CO- aufweist. In diesem Fall wird in der vorliegenden Anmeldung auch von einem Succinyl-Linker gesprochen.

Der Linker wird in einer Ausführungsform bevorzugt an eine OH Gruppe der Zuckerkomponente des Nucleosids oder an eine OH-Gruppe der zu einer Zuckerkomponente analogen Komponente des Nucleosidanalogons von R¹ kovalent gebunden. Im Falle einer Pentose kann es sich dabei z.B. um eine OH-Gruppe am 2'- oder 3'-C-Atom handeln. Grundsätzlich kommen aber auch OH-Gruppen an einer anderen Stelle des Nucleosids bzw. Nucleosidanalogons in Frage. OH-Gruppen oder gegebenenfalls andere funktionelle Gruppen, an denen keine Umsetzung stattfinden soll, können mit einer Schutzgruppe geschützt werden.

Alternativ oder auch zusätzlich kann der Linker (L) an die Basenkomponente bzw., bei Nucleosidanaloga, an die dazu analoge Komponente, gebunden werden. Beispielsweise kann der Linker an ein Stickstoffatom der Basenkomponente des Nucleosids oder der zu einer Basenkomponente analogen Komponente des Nucleosidanalogons von R¹ kovalent gebunden werden. Im Falle eines Linkers an der Basenkomponente bzw. dem Basenänalogon wird vorzugsweise ein Benzoyl-Linker, z.B. der Formel -OC-C₆H₄-, verwendet, wobei die Bindung an R¹ vorzugsweise über die funktionelle Gruppe -OC- erfolgt. Dies kann beispielsweise mittels eines entsprechenden Säurechlorids, wie nachfolgend abgebildet, erfolgen:

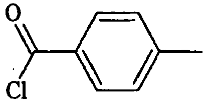

In einer bevorzugten Ausführungsform enthält das Nucleosid oder Nucleosidanalogon eine Pentose und der Linker, der hier ein Succinyl-Linker ist, wird an der 3'-Position der Pentose kovalent gebunden. In einem solchen Fall wird als Verbindung (III) bevorzugt eine Verbindung gemäß der folgenden allgemeinen Formel (IIIb) 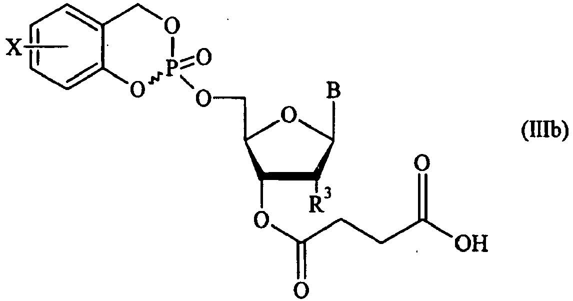 verwendet, wobei B ein Heterozyklus, vorzugsweise ein stickstoffhaltiger Heterozyklus, ist und R³ H, OH oder eine Schutzgruppe (SG) ist.

In einer bevorzugten Ausführungsform ist B eine der Nucleobasen Guanin, Adenin, Cytosin, Thymin oder Uracil. B kann aber auch beispielsweise ein Nucleobasenanalogon sein.

Als Festphase kommen beliebige Festphasen, d.h. unter den gewählten Reaktionsbedingungen im Wesentlichen unlösliche Verbindungen, z.B. Polymere, in Frage. Bevorzugte Festphasen weisen mindestens eine freie Aminogruppe -NH₂, vorzugsweise eine Vielzahl freier Aminogruppen, auf. Dies ist, z.B. im Falle eines Benzoyl-Linkers, jedoch nicht unbedingt erforderlich. In einer besonders bevorzugten Ausführungsform des erfindungsgemäßen Verfahrens handelt es sich bei der Festphase um ein Polystyrol (PS). Eine Aminogruppen aufweisende bevorzugte Festphase ist beispielsweise Aminomethylpolystyrol (AMPS).

Das Nucleophil ist vorzugsweise ausgewählt aus der Gruppe, bestehend aus Phosphat, Pyrophosphat, Glycosylphosphat, Nucleosid, Nucleosidmonophosphat, Nucleosiddiphosphat, Nucleosidtriphosphat Nucleosidanalogon, Nucleosidmonophosphatanalogon, Nucleosiddiphosphatanalogon, Nucleosidtriphosphatanalogon und α-deprotoniertem Glykosyl, Aminen, Aminosäuren, oder Salzen, vorzugsweise pharmazeutisch annehmbaren Salzen davon.

Im Falle des Phosphats lassen sich beispielsweise Nucleosiddiphosphate, im Falle des Pyrosphosphats Nucleosidtriphosphate herstellen, Verbindungen, die von großer Bedeutung sind. Die Verwendung von Glycosylphosphaten führt zu XDP-Zuckern, eine ebenfalls sehr bedeutsame Verbindungsklasse.

Dinucleosid-Polyphosphate gemäß der obigen Formel (V) ergeben sich als Produkt beim Einsatz von Nucleosidmono-, -di- und -triphosphat als Nucleophil. Dinucleotide wie beispielsweise NAD oder FAD lassen sich herstellen, indem die entsprechenden Nucleosidmonophosphate als Nucleophil eingesetzt werden. Verbindungen wie Diadenosintetraphosphat sind herstellbar unter Verwendung von Adenosintriphosphat (ATP). Neben einer 5'-5'-Verknüpfung der Nucleoside ist beispielsweise auch eine 5'-3'- oder eine 3'-3'-Verbrückung der Nucleoside durch die Phosphatgruppen möglich. Dafür müssen lediglich die eingesetzten Edukte entsprechend gewählt werden.

Der Einsatz von beispielsweise α-deprotoniertem Glykosyl als Nucleophil führt zu Zucker-Nucleotid-Biokonjugaten.

In einer besonders bevorzugten Ausführungsform des erfindungsgemäßen Verfahrens ist das Nucleosid in Verbindung (II) ausgewählt aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methyl-inosin, Guanosin, N²-2,2-Dimethylguanosin, N²-2-Methyl-guanosin, 7⁺-Methyl-guanosin, 2'-O-Methyl-guanosin.

Nucleoside oder Nucleosidanaloga sowie Nucleosidmono-, -di- und triphosphate oder Mono-, Di- und Triphosphate von Nucleosidanaloga können sowohl in der Verbindung gemäß Formel (II) als auch in dem Nucleophil bzw. als Nucleophil eingesetzt werden. Ein als Nucleophil eingesetztes Nucleosid kann beispielsweise eines der oben für die Verbindung (II) aufgeführten Nucleoside sein. Für den Fall, dass ein Nucleophil-Nucleosid bzw. -Nucleosidanalogon, d.h. ein als Nucleophil vorgesehenes Nucleosid oder Nucleosidanalogon bzw. deren Phosphate, eingesetzt wird, können das Nucleosid in Verbindung (II) und das Nucleophil-Nucleosid gleich oder auch verschieden sein, sind jedoch vorzugsweise verschieden.

Bei dem erfindungsgemäßen Verfahren wird der Linker wieder von R¹ abgespalten. Auf diese Weise wird das Konjugat gemäß der allgemeinen Formel (I), gegebenenfalls nach geeigneten Reinigungsschritten, freigesetzt. Die Abspaltung des Linkers kann beispielsweise durch Einwirkung von NH₃, z.B. in Form einer NH₃-Lösung, erfolgen.

Besonders bevorzugt wird das Verfahren unter einem geeigneten Inertgas, beispielsweise Stickstoffgas, durchgeführt.

Die Erfindung betrifft in einem weiteren Aspekt auch eine Verbindung der allgemeinen Formel (IIIa) 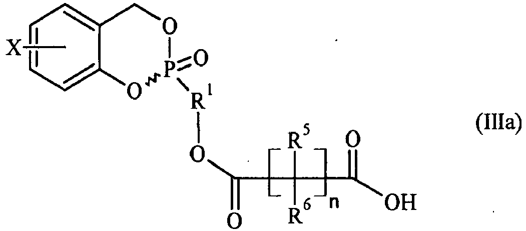 wobei die Verbindung (IIIa) ein oder mehrfach mit X substituiert sein kann,
X ein Elektronenakzeptor ist,
R¹ Nucl ist, wobei Nucl für Nucleosid oder Nucleosidanalogon steht,
n = 1, 2, 3, 4, 5, 6, 7, 8, 9 oder 10 ist und
R⁵ und R⁶, jeweils unabhängig voneinander, H, substituierte oder unsubstituierte Alkyl- oder Arylreste sind.

Bevorzugt handelt es bei der erfindungsgemäßen Vebindung um eine Verbindung der allgemeinen Formel (IIIb) 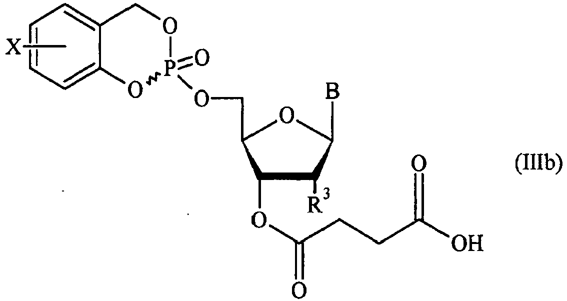 wobei B ein Heterozyklus, vorzugsweise stickstoffhaltiger Heterozyklus und R³ H, OH oder eine Schutzgruppe ist.

X ist, bei mehreren Substituenten X jeweils unabhängig voneinander, bevorzugt ausgewählt aus der Gruppe, bestehend aus H, Me, OMe, MeSO2, Keton, Formyl, Ester, C=0, COOH, NO₂ und Halogen.

Bevorzugt ist B eine der Nucleobasen Guanin, Adenin, Cytosin, Thymin oder Uracil. B kann aber auch beispielsweise ein Nucleobasenanalogon sein.

Nucl ist bevorzugt ausgewählt aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methyl-inosin, Guanosin, N²-2,2-Dimethyl-guanosin, N²-2-Methyl-guanosin, 7⁺-Methyl-guanosin und 2'-O-Methyl-guanosin.

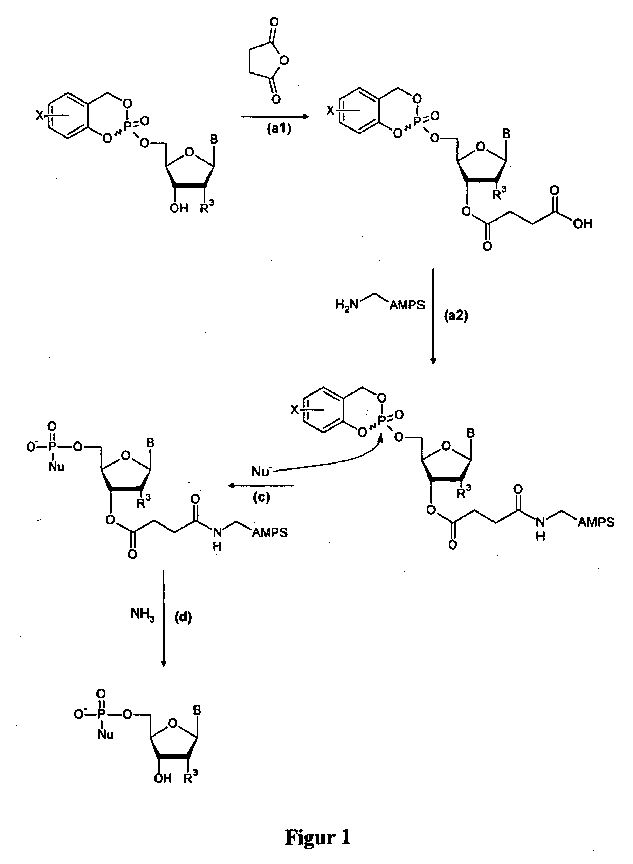

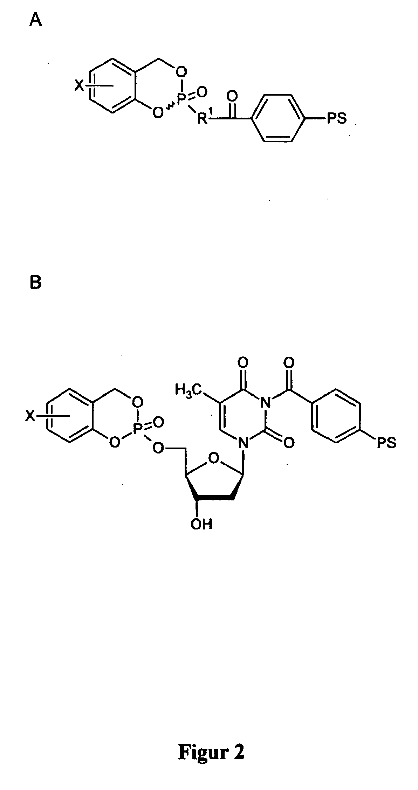

Die Erfindung wird im Folgenden anhand eines Ausführungsbeispiels und der Figuren 1 und 2 näher erläutert.

Figur 1 Schematische Darstellung einer bevorzugten Ausführungsform des erfindungsgemäßen Verfahrens.

Figur 2 Beispielhafte Darstellung von über einen Benzoyl-Linker an die Festphase PS gekoppelten Verbindungen (II). Figur 2A zeigt eine allgemeine Struktur, Figur 2B ein entsprechendes Thymidinderivat. PS = Polystyrol.

Figur 1 stellt schematisch eine bevorzugte Ausführungsform einer Alternative des erfindungsgemäßen Verfahrens dar. Zunächst wird im Schritt (a1) ein cycloSal-Nucleotid mit Bernsteinsäureanhydrid zur Reaktion gebracht, wodurch eine Verbindung gemäß Formel IIIb gebildet wird. Eine gegebenenfalls an der Pentose, z.B. Ribose, vorhandene OH-Gruppe an der 2'- Position wird dabei vorzugsweise mit einer Schutzgruppe geschützt. Der Verfahrensschritt addiert einen Succinyl-Linker gemäß Formel (IV) an das 3'-C-Atom der Pentose. Im darauf folgenden Schritt (a2) wird der Linker mit der Aminomethylpolystyrol(AMPS)-Festphase kovalent verbunden. Der Linker ist anschließend über eine Amidbindung an die Festphase AMPS gebunden. Im nächsten Schritt (c) erfolgt der Angriff des eingesetzten Nucleophils (Nu) auf das Phosphoratom des cycloSal-Phosphattriesters. Die Phenylphosphatesterbindung der cycloSal-Verbindung löst sich und das Nucleophil geht eine kovalente Bindung mit dem Phosphatatom ein. Anschließend wird durch eine spontane Reaktion das aus dem Nucleotid und dem Nucleophil bestehende Konjugat gebildet. In einem weiteren Schritt (d) wird das gebildete Konjugat, z.B. durch Einsatz von NH₃, von dem Linker abgespalten.

Bei den in Figur 2 dargestellten festphasengebundenen Beispielverbindungen (II) wird nicht AMPS als Festphase verwendet, sondern ein Polystyrol (PS). Der bzw. die Benzoyl-Linker is/sind an das Polystyrol gebunden.

Die nachfolgend beschriebenen Beispiele zeigen, dass es mit dem erfindungsgemäßen Verfahren möglich ist, phosphatverbrückte Nucleosid-Konjugate, insbesondere Nucleosiddi- und triphosphate, Nucleosiddiphosphat-Zucker und diphosphatverbrückte Nucleoside, in hoher Reinheit von 77-91 % bezüglich des immobilisierten cycloSal-Phosphattriesters zu synthetisieren. Ein deutlicher Vorteil gegenüber der Synthese dieser Verbindungen in Lösung ist beispielsweise die Möglichkeit, Reagenzien in großem Überschuss einsetzen zu können und dadurch hohe Ausbeuten zu erzielen. Des Weiteren ist es mittels dieses Verfahrens auch möglich, auch große Mengen eines gewünschten Produkts herzustellen, da die in Lösung problematische chromatographische Abtrennung der polaren Reagenzien entfällt. Das erfindungsgemäße Verfahren ist auf ein breites Spektrum von Verbindungen anwendbar und eröffnet den Weg zur Herstellung zahlreicher Zielmoleküle.

### Beispiel 1: Herstellung von Nucleosiddiphosphaten

Am Beispiel von Thymidindiphosphat wird zunächst die Herstellung von Nucleosiddiphosphaten beschrieben.

### 1.1 Verankerung eines cycloSal-Phosphattriesters mit einem basisch spaltbaren Linker

Die Reaktion wird unter Stickstoff als Inertgas durchgeführt. Ein nach etabliertem Verfahren (C. Meier; cycloSal Phosphates as Chemical Trojan Horses for Intracellular Nucleotide and Glycosylmonophosphate Delivery - Chemistry Meets Biology; Eur. J. Org. Chern. 2006, 1081-1102) synthetisierter 5-Chlor-cycloSal-phosphattriester von Thymidin 11 wird mit 1.5 Äq. Bernsteinsäureanhydrid zusammengefügt und zweimal mit abs. Acetonitril coevaporiert. Die Reagenzien werden in abs. Dichlormethan gelöst und mit 1 Äq. Diazabicycloundecan (DBU) versetzt. Nach vollständiger Umsetzung des Triesters (20-40 min) werden 2 Äq. Essigsäure zu dem Reaktionsgemisch gefügt und 10 min gerührt. Dann wird mit dest. Wasser und Dichlormethan extrahiert, die organischen Phasen vereinigt, über Natriumsulfat getrocknet und das Lösungsmittel unter vermindertem Druck entfernt. Der Rückstand wird am Chromatotron mit Dichlormethan und einem Methanol-Gradienten (+0,1 % Essigsäure) gereinigt. Die Reaktion verläuft quantitativ, doch die Abtrennung des einzigen Rückstandes, Überschuss von Bernsteinsäureanhydrid, am Chromatotron führt zu einer isolierten Ausbeute an 12 von 75%.

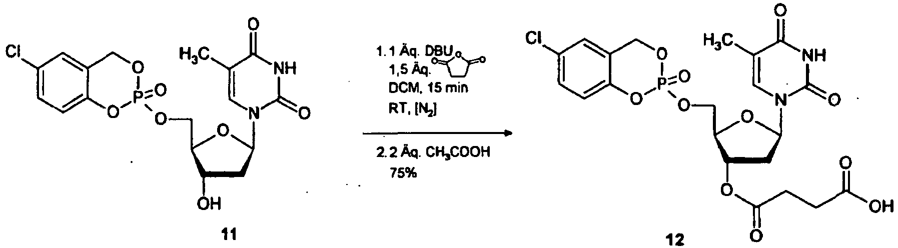

### 1.2 Festphasenreaktionen

Alle Reaktionen an der unlöslichen Festphase Aminomethylpolystyrol 13 (AMPS, 1,1 mmol/g, 100-200 mesh, Copolymer aus Styrol und 1% Divinylbenzol) 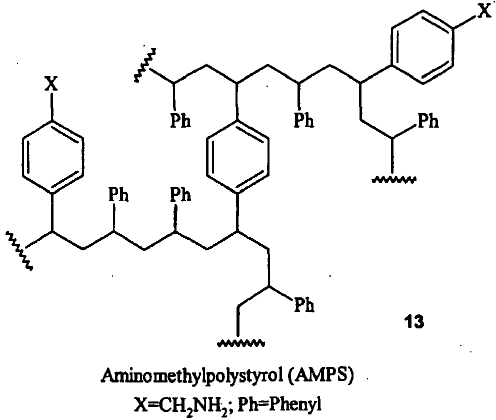 werden in einer Fritte und unter Stickstoff als Inertgas durchgeführt. Vor einer Reaktion wird das Harz im Vakuum getrocknet und, wenn für die Reaktion nötig, coevaporiert. Dann wird das Harz in abs. DMF für eine halbe Stunde gequollen (ca. 1 mL pro 0,1 mmol Beladung), um ein verbessertes Eindringen der Reagenzien in die Festphasenmatrix zu ermöglichen. Die Reaktionen werden auf einem Schüttelgerät geschüttelt. Die Waschvorgänge werden ebenfalls unter Stickstoff durchgeführt, wobei die dafür verwendeten Lösungsmittel nicht absolut sein müssen. Die Abspaltung vom Harz erfolgt mit 25%iger wässriger NH₃-Lösung für 2 h bei 50°C. Die Abspaltlösung wird entfernt und das Harz viermal mit insgesamt 50 mL H₂O gewaschen. Wasch- und Abspaltlösung werden vereinigt und gefriergetrocknet. Das an der Festphase immobilisierte Nucleosid, dessen Menge aufgrund des Kaiser-Tests bekannt ist (siehe unten), wird während der zweistündigen Abspaltung quantitativ von der Festphase abgespalten. Die in den folgenden Abschnitten genannten Reinheiten beziehen sich auf diese Rohprodukte. Wenn im Rückstand nach der Gefriertrocknung neben dem gewünschten Produkt das, bei unvollständiger Umsetzung des cycloSal-Phosphattriesters als Nebenprodukt entstehende, Nucleosid-5'-monophosphat enthalten ist, kann dieses durch anschließende RP18-Chromatographie entfernt werden.

### 1.3 Immobilisierung von cycloSal-Phosphattriestern an der Festphase

Die Immobilisierung des cycloSal-Phosphattriesters 12 an der Festphase beruht auf einer Amidbindung zwischen der Aminogruppe (=Ankergruppe) des Harzes Aminomethylpolystyrol (AMPS) 13 und der Carbonsäuregruppe des Linkers. Diese ist während aller weiteren Umsetzungen stabil, und der Linker verbleibt nach der Abspaltung am Harz. Für die Kupplung wird linkergebundener 5-Chlor-cycloSalphosphattriester 12 mit äquimolaren Mengen N,N'-Diisopropylcarbodiimid (DIC) und 1-Hydroxy-benzotriazol (HOBt) in absolutem DMF gelöst. Diese Lösung wird zu 0,75 Äq. in DMF gequollenem Harz Aminomethylpolystyrol 13 gegeben und bei Raumtemperatur geschüttelt. Die Reaktionszeit beträgt mindestens 2 Tage. Die Reaktion ist beendet, wenn alle Aminogruppen des Harzes belegt sind. Dies wird mit Hilfe des Kaiser-Tests, welcher auf der Reaktion von Ninhydrin mit freien primären Aminogruppen beruht, überprüft (V. K. Sarin, S. B. H. Kent; J. P. Tam, R. B. Merrifield; Quantitative monotoring of solid-phase peptide synthesis by the ninhydrin reaction; Anal. Biochern. 1981, 117, 147-157). Unter gleichen Kupplungsbedingungen erfolgte auch die Immobilisierung des 5-Chlor-cycloSal-phosphattriesters von BVdU 14 ((E)-5-(2-Bromvinyl)-2'-desoxyuridin, Brivudin, Zostex®).

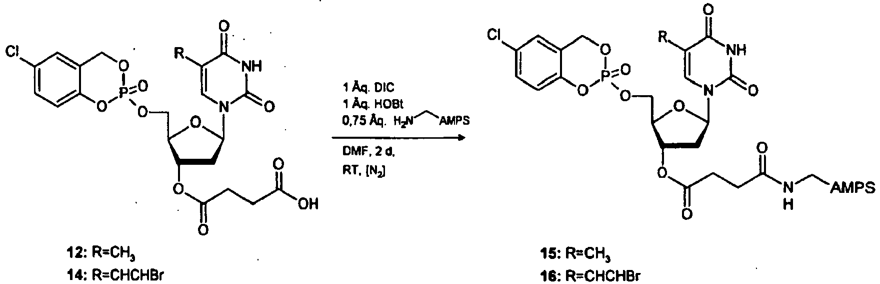

### 1.4 Festphasensynthese von Nucleosid-5'polyphosphaten

Für die Thymidindiphosphatsynthese wird zunächst das als Nucleophil verwendete Phosphat in der Tetra-n-butylammonium-Form synthetisiert, indem Phosphorsäure mit Tetra-n-butylammoniumhydroxid bis zu einem pH-Wert von 5 titriert wird. 6 Äquivalente des nach Gefriertrocknung als hygroskopisches Salz erhaltenen Phosphats werden in einer Stickstoffatmosphäre in absolutem DMF über Molsieb 4 Å mindestens 3 h getrocknet. Das mit cycloSal-Phosphattriester beladene Harz 15 wird in einer Fritte dreimal mit abs. MeCN coevaporiert und in abs. DMF gequollen. Darauf folgend wird das Phosphat zu dem Harz gegeben und 16 h bei RT geschüttelt. Nach Entfernen der Reaktionslösung wird das Harz in einer Stickstoffatmosphäre je viermal mit jeweils insgesamt 50 mL DMF, DCM und H₂O gewaschen und anschließend 0,5 h im Ölpumpenvakuum getrocknet. Nach quantitativer Abspaltung und Gefriertrocknung wird Thymidin-5'-diphosphat 17 mit einer Reinheit, bezogen auf den an der Festphase immobilisierten cycloSalPhosphattriester, von 90% erhalten.

BVdU-5'-diphosphat 18 wurde auf analoge Weise hergestellt, wobei 16 Äq. des Phosphat-Salzes verwendet und eine Reinheit von 91 % erzielt wurde.

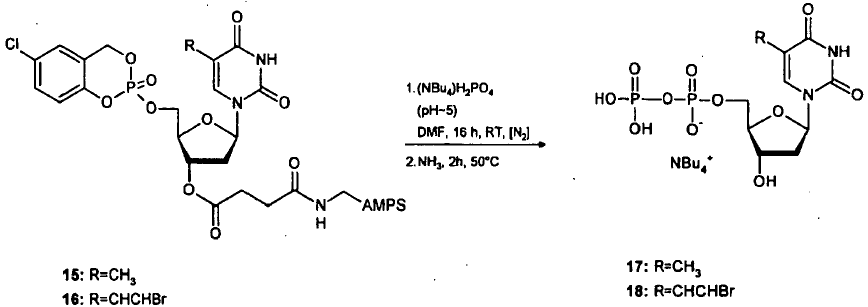

### Beispiel 2: Herstellung von Nucleosidtriphosphaten

Synthesen der Nucleosid-5'-triphosphate von Thymidin, 19, und BVdU, 20, wurden analog zu den Nucleosid-5'-diphosphaten mit Pyrophosphat als Nucleophil in der Tetra-n-butylammonium-Form und einem pH-Wert des Salzes von etwa 5 durchgeführt. Es wurden 4 Äquivalente des Salzes im Fall von Thymidin und 34 Äq. im Fall von BVdU verwendet und eine Reinheit, bezogen auf den an der Festphase immobilisierten cycloSal-Phosphattriester, an 19 von 89% und an 20 von 93% erzielt. Ein solch extrem hoher Überschuss an Nucleophil, wie er im Fall des BVdUs verwendet wurde, ist nicht unbedingt erforderlich. Er wurde hier eingesetzt, weil BVdU sehr teuer ist und durch den hohen Überschuss eine entsprechend hohe Ausbeute erwartet wurde. 4 Äquivalente Pyrophosphat sind jedoch normalerweise ausreichend für eine hohe Ausbeute an Nucleosid-5'-diphosphat.

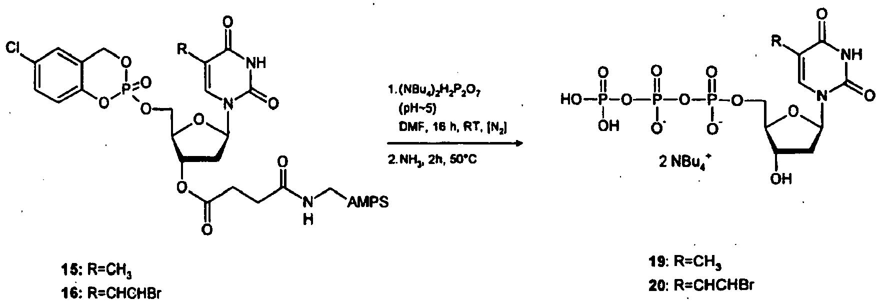

### Beispiel 3: Festphasensynthese von Dinucleosid-5'-polyphosphaten

Zunächst wird das als Nucleophil verwendete Uridin-5'-monophosphat mittels Ionenaustausch (Dowex 50X8) von der Dinatrium- in die Tetra-n-butylammonium-Form mit einem pH-Wert des Salzes von etwa 5 umgesalzen. 8 Äquivalente des nach Gefriertrocknung als hygroskopisches Salz erhaltenen UMPs werden in einer Stickstoffatmosphäre in absolutem DMF über Molsieb 4 Å mindestens 3 h getrocknet. Das mit cycloSal-Phosphattriester beladene Harz 15 wird in einer Fritte dreimal mit abs. MeCN coevaporiert und in abs. DMF gequollen. Anschließend wird UMP sowie einige Kömer aktiviertes Molsieb 4 Å zu dem Harz gegeben und 5 d bei RT geschüttelt. Nach Entfernen der Reaktionslösung wird das Harz in einer Stickstoffatmosphäre je viermal mit jeweils insgesamt 50 mL DMF, DCM und H₂O gewaschen und anschließend 0,5 h im Ölpumpenvakuum getrocknet. Nach quantitativer Abspaltung und Gefriertrocknung wird Up₂T 21 mit einer Reinheit, bezogen auf den an der Festphase immobilisierten cycloSal-Phosphattriester 15, von 77% erhalten.

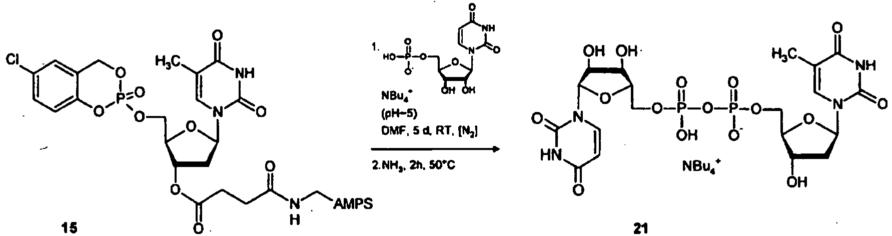

### Beispiel 4: Festphasensynthese von Nucleosid-5'-diphosphat-Zuckern

Das als Nucleophil verwendete peracetylierte ß-D-Glucopyranosyl-1-phosphat wird nach einem etabilierten Verfahren als Triethylammonium-Salz erhalten (J. W. Perich, R. B. Johns; A New Convenient and Efficient General Procedure for the Conversion of Alcohols into their Dibenzyl Phosphoramidite; Tetrahedron Lett. 1987, 28, 101-102). Dieses wird per Ionenaustausch (Dowex 50X8) in die Tetra-n-butylammonium-Form mit einem pH-Wert des Salzes von etwa 5 umgesalzen und gefriergetrocknet. 9 Äq. des danach erhaltenen Salzes werden 1 d im Ölpumpenvakuum getrocknet und 6 h in absolutem DMF über Molsieb 4 A getrocknet. Das mit cycloSal-Phosphattriester beladene Harz 15 wird in einer Fritte dreimal mit abs. MeCN coevaporiert und in abs. DMF gequollen. Anschließend wird das Zuckerphosphat zu dem Harz gegeben und 6 d bei RT geschüttelt. Nach Entfernen der Reaktionslösung wird das Harz in einer Stickstoffatmosphäre je viermal mit jeweils insgesamt 200 mL DMF und 40 mL DCM gewaschen und anschließend 0.5 h im Ölpumpenvakuum getrocknet. Nach Abspaltung und Gefriertrocknung wird deacetylierte Thymidin-5'-diphosphat-ß-D-Glucopyranose 22 mit einer Reinheit, bezogen auf den an der Festphase immobilisierten cycloSal-Phosphattriester 15, von 78% erhalten. Chromatographische Reinigung an RP18-Kieselgel ermöglicht die Entfernung des zu 16% als Nebenprodukt entstandenen Nucleosid-5'-monophosphats sowie der restlichen 6% Verunreinigungen. Das im Überschuss verwendete Zuckerphosphat kann durch Aufarbeitung der Reaktionslösung wiedergewonnen werden. Dazu wird diese vom Lösungsmittel befreit und der Rückstand chromatographiert (MeCN/H₂O 7:3).

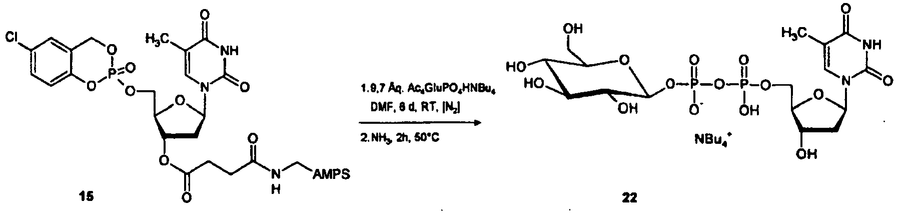

## Patentansprüche

1. Verfahren zur festphasengestützten Herstellung einer Verbindung der allgemeinen Formel (I): 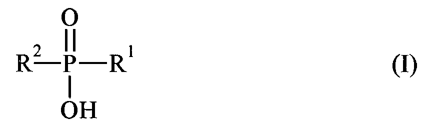 oder eines Salzes davon, wobei
R¹ Nucl ist, wobei Nucl für Nucleosid oder Nucleosidanalogon steht,
R² eine organische Verbindung oder Phosphat oder Pyrophosphat ist, umfassend die Schritte des:
a) Addierens eines Linkers (L) an eine Verbindung der allgemeinen Formel (II) 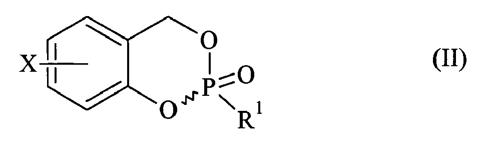 wobei R¹ wie oben definiert ist und die Verbindung (II) ein oder mehrfach mit X substituiert sein kann, und wobei X ein Elektronenakzeptor, H oder OMe ist, wobei Me für Methyl steht,
zur Herstellung einer Verbindung der Formel (III) 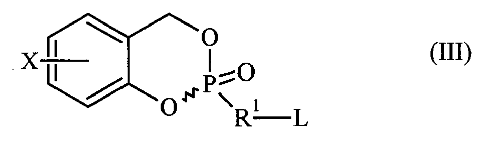 wobei X, und R¹ wie oben definiert sind und L=Linker ist, und Immobilisierens der Verbindung (III) durch kovalente Bindung des Linkers (L) an eine mindestens eine Aminogruppe -NH₂ enthaltende Festphase, oder
b) Addierens eines kovalent an eine Festphase gebundenen Linkers (L) an die Verbindung der allgemeinen Formel (II),
c) Umsetzens des Produktes aus Schritt a) oder b) mit einem mit R² identischen oder R² umfassenden Nucleophil, und
d) Abspaltens des Linkers (L) vom Rest R¹.

2. Verfahren nach Anspruch 1, **dadurch gekennzeichnet, dass** in Schritt a) ein Linker (L) gemäß der allgemeinen Formel (IV) 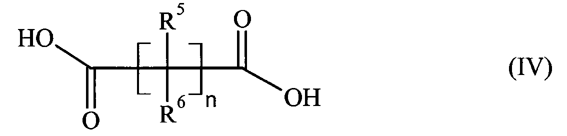 verwendet und eine Verbindung der allgemeinen Formel (IIIa) 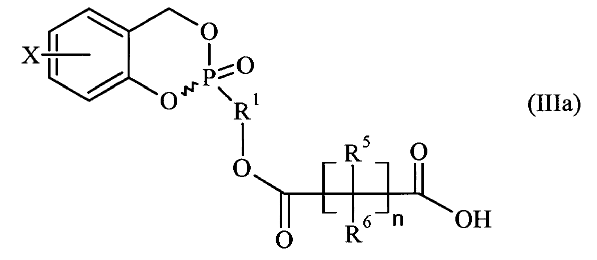 hergestellt wird, wobei R⁵ und R⁶, jeweils unabhängig voneinander, H, substituierte oder unsubstituierte Alkyl- oder Arylreste sind, wobei Alkyl geradkettige und verzweigtkettige Alkylgruppen, Cykloalkylgruppen, alkylsubstituierte Cycloalkylgruppen, cycloalkylsubstituierte Alkylgruppen sowie Alkylgruppen, die Sauerstoff-, Stickstoff-, Schwefel- oder Phosphoratome aufweisen, die ein oder mehrere Kohlenstoffatome des Kohlenwasserstoffgerüsts ersetzen, beinhaltet, und wobei Aryl Gruppen mit Aromatizität bedeutet, einschließlich Heteroarylen, 5- und 6-gliedriger aromatischer Einzelringgruppen, die null bis vier Heteroatome beinhalten können, und multizyklischer Systeme mit mindestens einem aromatischen Ring, und n = 1, 2, 3, 4, 5, 6, 7, 8, 9 oder 10, vorzugsweise 2, ist.

3. Verfahren nach einem der vorhergehenden Ansprüche, **dadurch gekennzeichnet, dass** der Linker (L) an eine OH Gruppe der Zuckerkomponente des Nucleosids oder an eine zu einer Zuckerkomponente analogen Komponente des Nucleosidanalogons von R¹ kovalent gebunden wird.

4. Verfahren nach Anspruch 3, **dadurch gekennzeichnet, dass** in Schritt a) als Verbindung (III) eine Verbindung gemäß der folgenden allgemeinen Formel (IIIb) 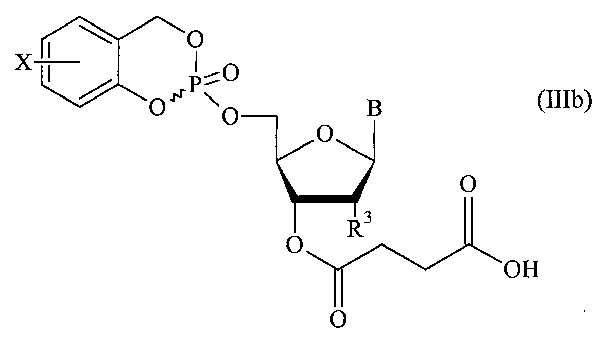 verwendet wird, wobei B ein Heterozyklus, vorzugsweise stickstoffhaltiger Heterozyklus, besonders bevorzugt eine der Nucleobasen Guanin, Adenin, Cytosin, Thymin oder Uracil, ist und R³ H, OH oder eine Schutzgruppe ist.

5. Verfahren nach Anspruch 1, **dadurch gekennzeichnet, dass** der Linker (L) an ein Stickstoffatom der Basenkomponente des Nucleosids oder ein Stickstoffatom einer zu einer Basenkomponente analogen Komponente des Nucleosidanalogons von R¹ kovalent gebunden wird, wobei der Linker (L) vorzugsweise ein Benzoyl-Linker der Formel -OC-C₆H₄- ist und die Bindung an R¹ über die funktionelle Gruppe -OC- erfolgt.

6. Verfahren nach einem der vorhergehenden Ansprüche, **dadurch gekennzeichnet, dass** als Festphase Polystyrol, bevorzugt Aminomethylpolystyrol, verwendet wird.

7. Verfahren nach einem der vorhergehenden Ansprüche, **dadurch gekennzeichnet, dass** X, bei mehreren Substituenten X unabhängig voneinander, ausgewählt ist aus der Gruppe, bestehend aus H, Me, OMe, MeSO₂, Keton, Formyl, Ester, C=O, COOH, NO₂ und Halogen, wobei Me für Methyl steht.

8. Verfahren nach einem der vorhergehenden Ansprüche, **dadurch gekennzeichnet, dass**
- das Nucleophil ausgewählt ist aus der Gruppe, bestehend aus Phosphat, Pyrophosphat, Glycosylphosphat, Nucleosid, Nucleosidmonophosphat, Nucleosiddiphosphat, Nucleosidtriphosphat, Nucleosidanalogon, Nucleosidmonophosphatanalogon, Nucleosiddiphosphatanalogon, Nucleosidtriphosphatanalogon,α-deprotoniertem Glykosyl, Aminen, Aminosäuren, oder Salzen davon, und/oder
- Nucl ausgewählt ist aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methyl-inosin, Guanosin, N²-2,2-Dimethylguanosin, N²-2-Methyl-guanosin, 7⁺-Methyl-guanosin und 2'-O-Methylguanosin.

9. Verfahren nach Anspruch 8, **dadurch gekennzeichnet, dass** das Nucleosid oder Nucleosidanalogon Nucl in Verbindung (II) und das Nucleophil-Nucleosid oder Nucleophil-Nucleosidanalogon gleich oder verschieden, vorzugsweise verschieden sind.

10. Verfahren nach einem der vorhergehenden Ansprüche, **dadurch gekennzeichnet, dass** das Verfahren unter Inertgasatmosphäre, vorzugsweise unter Stickstoffgas durchgeführt wird.

11. Verbindung der allgemeinen Formel (IIIa) 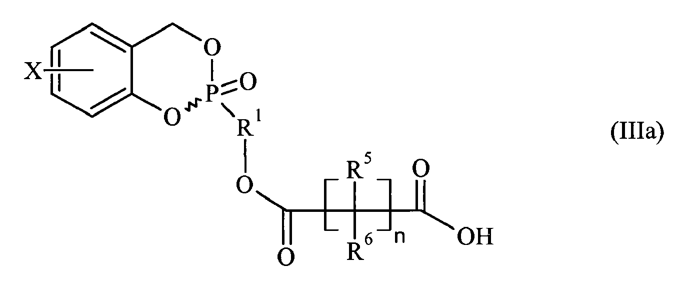 wobei die Verbindung (IIIa) ein oder mehrfach mit X substituiert sein kann,
X ein Elektronenakzeptor, H oder OMe ist, wobei Me für Methyl steht,
R¹ Nucl ist, wobei Nucl für Nucleosid oder Nucleosidanalogon steht,
n = 1, 2, 3, 4, 5, 6, 7, 8, 9 oder 10 ist und
R⁵ und R⁶, jeweils unabhängig voneinander, H, substituierte oder unsubstituierte Alkyl- oder Arylreste sind, wobei Alkyl geradkettige und verzweigtkettige Alkylgruppen, Cykloalkylgruppen, alkylsubstituierte Cycloalkylgruppen, cycloalkylsubstituierte Alkylgruppen sowie Alkylgruppen, die Sauerstoff-, Stickstoff-, Schwefel- oder Phosphoratome aufweisen, die ein oder mehrere Kohlenstoffatome des Kohlenwasserstoffgerüsts ersetzen, beinhaltet, und wobei Aryl Gruppen mit Aromatizität bedeutet, einschließlich Heteroarylen, 5- und 6-gliedriger aromatischer Einzelringgruppen, die null bis vier Heteroatome beinhalten können, und multizyklischer Systeme mit mindestens einem aromatischen Ring.

12. Verbindung nach Anspruch 11, **dadurch gekennzeichnet, dass** es sich um eine Verbindung der allgemeinen Formel (IIIb) 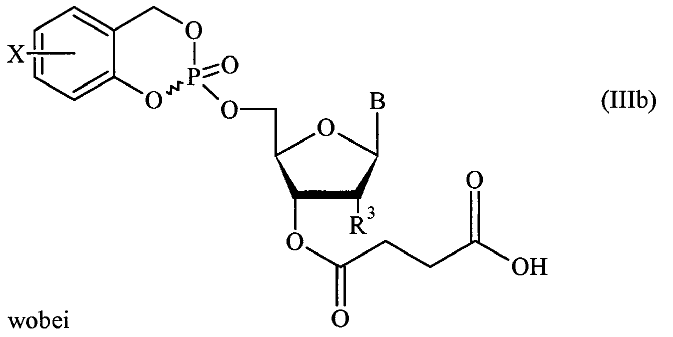 handelt, wobei
B ein Heterozyklus, vorzugsweise stickstoffhaltiger Heterozyklus, besonders bevorzugt eine der Nucleobasen Guanin, Adenin, Cytosin, Thymin oder Uracil, ist, und
R³ H, OH oder eine Schutzgruppe ist.

13. Verbindung nach Anspruch 11 oder 12, **dadurch gekennzeichnet, dass** X, bei mehreren Substituenten X unabhängig voneinander, ausgewählt ist aus der Gruppe, bestehend aus H, Me, OMe, MeSO₂, Keton, Formyl, Ester, C=O, COOH, NO₂ und Halogen, wobei Me für Methyl steht.

14. Verbindung nach Anspruch 11, **dadurch gekennzeichnet, dass** Nucl ausgewählt ist aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methyl-inosin, Guanosin, N²-2,2-Dimethyl-guanosin, N²-2-Methyl-guanosin, 7⁺-Methyl-guanosin und 2'-O-Methyl-guanosin.

## Claims

1. A method for the solid-phase based production of a compound of the general formula (I): 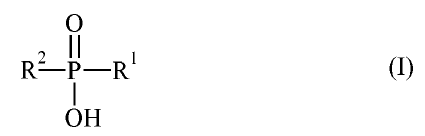 or a salt thereof, wherein
R¹ is Nucl, wherein Nucl is nucleoside or nucleoside analog,
R² is an organic compound or phosphate or pyrophosphate, comprising the steps of:
a) adding a linker (L) to a compound of the general formula (II) 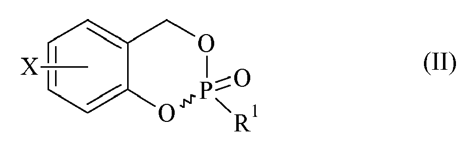 wherein R¹ is as defined above and the compound (II) may be substituted one or more times with X, X being an electron acceptor, H or OMe, wherein Me is methyl,
for the production of a compound of the formula (III) 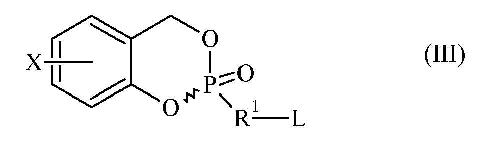 wherein X and R¹ are as defined above and L=linker, and immobilizing the compound (III) by covalently binding the linker (L) to a solid phase having at least one amino group -NH₂, or
b) adding a linker (L) covalently bound to a solid phase to the compound according to the general formula (II),
c) reacting the products from step a) or b) with a nucleophile identical to R² or comprising R², and
d) releasing the linker (L) from the residue R¹.

2. The method according to claim 1, **characterized in that** in step a) a linker (L) according to the general formula (IV) 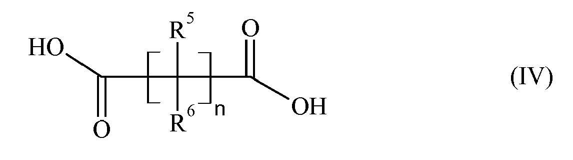 is used and a compound of the general formula (IIIa) 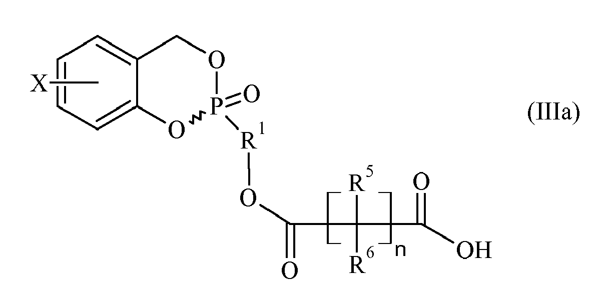 is produced, wherein R⁵ and R⁶ are, each independent from each other, H, substituted or unsubstituted alkyl or aryl residues, alkyl including straight-chained and branch-chained alkyl groups, cycloalkyl groups, alkyl-substituted cycloalkyl groups, cycloalkyl-substituted alkyl groups as well as alkyl groups having oxygen, nitrogen, sulfur or phosphor atoms replacing one or more carbon atoms of the hydrocarbon backbone, and aryl meaning groups with aromaticity, including heteroaryls, 5- and 6-membered aromatic single-ring groups, which may contain zero to four heteroatoms, and multi-cyclic systems with at least one aromatic ring, and wherein n is 1, 2, 3, 4, 5, 6, 7, 8, 9 or 10, preferably 2.

3. The method according to one of the preceding claims, **characterized in that** the linker (L) is covalently bound to an OH group of the sugar component of the nucleoside or to a component analogous to a sugar component of the nucleoside analog of R¹.

4. The method according to claim 3, **characterized in that** in step a) a compound according to the following general formula (IIIb) 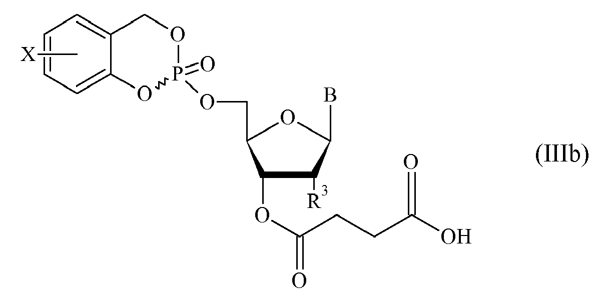 is used as compound (III), wherein B is a heterocycle, preferably a nitrogen containing heterocycle, especially preferably one of the nucleobases guanine, adenine, cytosine, thymine or uracil, and wherein R³ is H, OH or a protecting group.

5. The method according to claim 1, **characterized in that** the linker (L) is covalently bound to a nitrogen atom of the base component of the nucleoside or a nitrogen atom of a component analogous to a base component of the nucleoside analog of R¹, wherein the linker (L) preferably is a benzoyl linker of the formula -OC-C₆H₄-, and wherein the bond to R¹ occurs via the functional group -OC-.

6. The method according to one of the preceding claims, **characterized in that** polystyrene, preferably aminomethyl polystyrene, is used as a solid phase.

7. The method according to one of the preceding claims, **characterized in that** X, in case of multiple substituents X independently from each other, is selected from the group consisting of H, Me, OMe, MeSO₂, ketone, formyl, ester, C=O, COOH, NO₂ and halogen, Me being methyl.

8. The method according to one of the preceding claims, **characterized in that**
- the nucleophile is selected from the group consisting of phosphate, pyrophosphate, glycosyl phosphate, nucleoside, nucleoside monophosphate, nucleoside diphosphate, nucleoside triphosphate, nucleoside analog, nucleoside monophosphate analog, nucleoside diphosphate analog, nucleoside triphosphate analog, α-deprotonated glycosyl, amines, amino acids, or salts thereof, and/or
- Nucl is selected from the group consisting of adenosine, guanosine, cytidine, thymidine, uridine, deoxyadenosine, deoxyguanosine, inosine, deoxycytidine, deoxyuridine, deoxythymidine, 2-thiocytidine, N⁴-acetyl-cytidine, 2'-o-methyl-cytidine, 3-methyl-cytidine, 5-methyl-cytidine, 2-thiouridine, pseudouridine, dihydrouridine, 5-(carboxy-hydroxymethyl)-uridine, 5-carboxymethylaminomethyl-uridine, 5-methylaminomethyl-uridine, 5-methoxy-carbonylmethyl-uridine, 5-methoxy-uridine, ribothymidine, 1-methyl-adenosine, 2-methyl-adenosine, N⁶-methyl-adenosine, inosine, 1-methyl-inosine, guanosine, N²-2,2-dimethyl-guanosine, N²-2-methyl-guanosine, 7⁺-methyl-guanosine and 2'-O-methylguanosine.

9. The method according to claim 8, **characterized in that** the nucleoside or nucleoside analog Nucl in compound (II) und the nucleophile nucleoside or nucleophile nucleoside analog are the same or different, preferably different.

10. The method according to one of the preceding claims, **characterized in that** the method is carried out under inert gas atmosphere, preferably under nitrogen gas.

11. A compound of the general formula (IIIa) 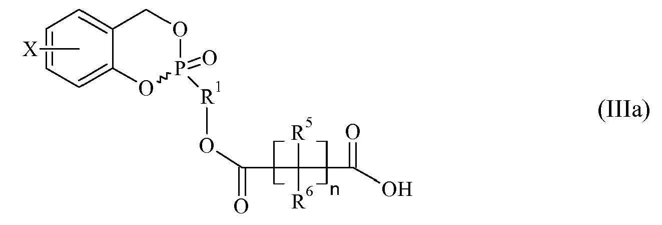 wherein the compound (IIIa) may be substituted one or more times with X,
X is an electron acceptor, H or OMe, wherein Me is methyl,
R¹ is Nucl, wherein Nucl stands for nucleoside or nucleoside analog,
n is 1, 2, 3, 4, 5, 6, 7, 8, 9 or 10 and
R⁵ and R⁶ are, each independently from each other, H, substituted or unsubstituted alkyl or aryl residues, alkyl including straight-chained and branch-chained alkyl groups, cycloalkyl groups, alkyl-substituted cycloalkyl groups, cycloalkyl-substituted alkyl groups as well as alkyl groups having oxygen, nitrogen, sulfur or phosphor atoms replacing one or more carbon atoms of the hydrocarbon backbone, and aryl meaning groups with aromaticity, including heteroaryls, 5- and 6-membered aromatic single-ring groups, which may contain zero to four heteroatoms, and multi-cyclic systems with at least one aromatic ring.

12. The compound according to claim 11, **characterized in that** the compound is a compound of the general formula (IIIb), 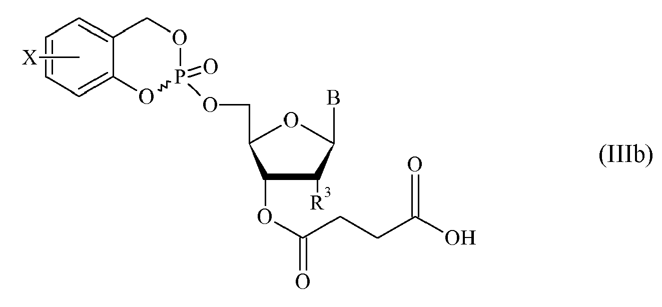 wherein
B is a heterocycle, preferably a nitrogen containing heterocycle, especially preferably one of the nucleobases guanine, adenine, cytosine, thymine or uracil, and
R³ is H, OH or a protecting group.

13. The compound according to claim 11 or 12, **characterized in that** X, in case of multiple substituents X independently from each other, is selected from the group consisting of H, Me, OMe, MeSO₂, ketone, formyl, ester, C=O, COOH, NO₂ and halogen, Me being methyl.

14. The compound according to claim 11 , **characterized in that** Nucl is selected from the group consisting of adenosine, guanosine, cytidine, thymidine, uridine, deoxyadenosine, deoxyguanosine, inosine, deoxycytidine, deoxyuridine, deoxythymidine, 2-thiocytidine, N⁴-acetyl-cytidine, 2'-O-methyl-cytidine, 3-methyl-cytidine, 5-methyl-cytidine, 2-thiouridine, pseudouridine, dihydrouridine, 5-(carboxyhydroxymethyl)-uridine, 5-carboxymethylaminomethyl-uridine, 5-methylaminomethyl-uridine, 5-methoxy-carbonylmethyl-u of uridine, 5-methoxy-uridine, ribo-thymidine, 1-methyl-adenosine, 2-methyl-adenosine, N⁶-methyl-adenosine, inosine, 1-methyl-inosine, guanosine, N²-2,2-dimethyl-guanosine, N²-2-methyl-guanosine, 7⁺-methyl-guanosine and 2'-O-methyl-guanosine.

## Revendications

1. Procédé de préparation sur phase solide d'un composé répondant à la formule générale (I) : 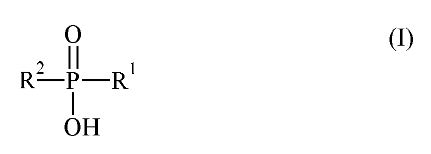 ou d'un de ses sels,
R¹ étant Nucl, Nucl signifiant nucléoside ou analogue de nucléoside,
R² étant un composé organique ou un phosphate ou pyrophosphate,
comprenant les étapes consistant à :
a) réaliser unz réaction d'addition entre un groupe de liaison (L) et un composé répondant à la formule générale (II) 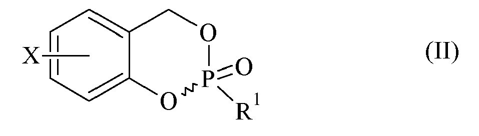 R¹ étant défini comme ci-dessus et le composé (II) pouvant comporter un ou plusieurs substituants X, et X étant un accepteur d'électrons, un H ou un groupe OMe, et Me représentant méthyle,
pour ainsi préparer un composé répondant à la formule (III) 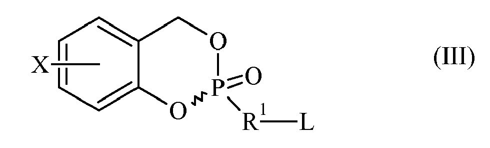 X et R¹ étant définis comme ci-dessus et L étant un groupe de liaison, puis immobiliser le composé (III) en établissant une liaison covalente entre le groupe de liaison (L) et au moins une phase solide contenant au moins un groupe amino -NH₂, ou
b) réaliser une réaction d'addition entre un groupe de liaison (L), lié de manière covalente à une phase solide, et un composé répondant à la formule générale (II),
c) faire réagir le produit issu de l'étape a) ou b) avec un nucléophile correspondant à R² ou comprenant R², et
d) séparer le groupe de liaison (L) du radical R¹.

2. Procédé selon la revendication 1, **caractérisé en ce que** l'on utilise à l'étape a) un groupe de liaison (L) selon la formule générale (IV) 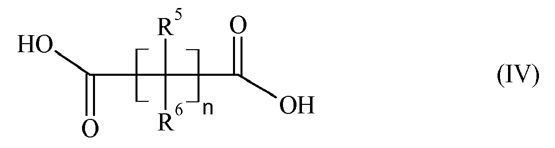 pour préparer un composé répondant à la formule générale (IIIa) 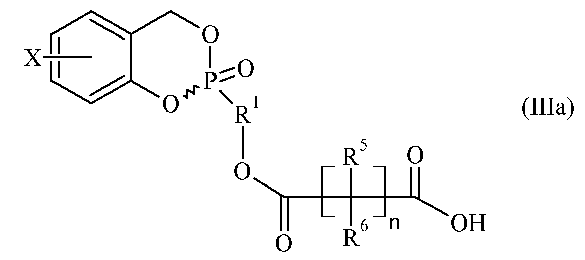 R⁵ et R⁶ représentant, indépendamment l'un de l'autre, H ou des radicaux alkyle ou aryle substitués ou non-substitués, alkyle comprenant des groupes alkyle linéaires et ramifiés, des groupes cycloalkyle, des groupes cycloalkyle pourvus de substituants alkyle, des groupes alkyle pourvus de substituants cycloalkyle ainsi que des groupes alkyle comportant des atomes d'oxygène, d'azote, de soufre ou de phosphore remplaçant un ou plusieurs atomes de carbone du squelette d'hydrocarbure, et aryle désignant des groupes à caractère aromatique y compris les hétéroaryles, les groupes monocycliques aromatiques constitués de 5 à 6 chaînons pouvant contenir zéro à quatre hétéroatomes, et les systèmes polycycliques comportant au moins un cycle aromatique, et n étant égal à 1, 2, 3, 4, 5, 6, 7, 8, 9 ou 10, dé préférence à 2.

3. Procédé selon l'une des revendications précédentes, **caractérisé en ce que** le groupe de liaison (L) est attaché par liaison covalent à R¹, et plus précisément à un groupe OH de la partie sucre du nucléoside ou à une partie dudit analogue de nucléoside laquelle est analogue à une partie sucre.

4. Procédé selon la revendication 3, **caractérisé en ce que** l'on utilise à l'étape a) en tant que compose (III) un composé selon la formule générale (IIIb) suivante 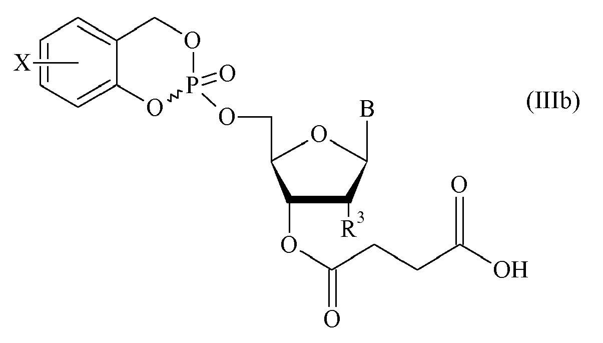 B étant un hétérocycle, de préférence un hétérocycle azoté, s'agissant avec une préférence particulière d'une des nucléobases guanine, adénine, cytosine, thymine ou uracile, et R³ représentant H, OH ou un groupe protecteur.

5. Procédé selon la revendication 1, **caractérisé en ce que** le groupe de liaison (L) est attaché par liaison covalente à R¹, et plus précisément à un atome d'azote de la partie base du nucléoside ou à un atome d'azote d'une partie dudit analogue de nucléoside laquelle est analogue à une partie base, le groupe de liaison (L) étant de préférence un groupe de liaison benzoyle répondant à la formule -OC-C₆H₄-, et liaison avec R¹ étant établie par l'intermédiaire du groupe fonctionnel -OC-.

6. Procédé selon l'une des revendications précédentes, **caractérisé en ce que** l'on utilise, en tant que phase solide, du polystyrène, préférentiellement de l'aminométhylpolystyrène.

7. Procédé selon l'une des revendications précédentes, **caractérisé en ce que** X est choisi dans le groupe constitué de H, Me, OMe, MeSO₂, cétone, formyle, ester, C=O, COOH, NO₂ et halogène, Me représentant méthyle, et chaque X pouvant être choisi indépendamment de l'autre si plusieurs substituants de ce type sont présents.

8. Procédé selon l'une des revendications précédentes, **caractérisé en ce que**
- ledit nucléophile est choisi dans le groupe constitué de phosphate, pyrophosphate, glycosylphosphate, nucléoside, monophosphate d'un nucléoside, diphosphate d'un nucléoside, triphosphate d'un nucléoside, analogue d'un nucléoside, analogue d'un monophosphate d'un nucléoside, analogue d'un diphosphate d'un nucléoside, analogue d'un triphosphate d'un nucléoside, glycosyle α-déprotoné, amines, acides aminés, ou leurs sels, et/ou
- Nucl est choisi dans le groupe constitué d'adénosine, guanosine, cytidine, thymidine, uridine, désoxyadénosine, désoxyguanosine, inosine, désoxycytidine, désoxyuridine, désoxythymidine, 2-thiocytidine, N⁴-acétyl-cytidine, 2'-O-méthyl-cytidine, 3-méthyl-cytidine, 5-méthyl-cytidine, 2-thiouridine, pseudouridine, dihydrouridine, 5-(carboxyhydroxymethyl)-uridine, 5-carboxymethylaminomethyl-uridine, 5-méthylaminomethyl-uridine, 5-méthoxy-carbonylmethyl-uridine, 5-méthoxy-uridine, ribo-thymidine, 1-méthyl-adénosine, 2-méthyl-adénosine, N⁶-méthyl-adénosine, inosine, 1-méthyl-inosine, guanosine, N²-2,2-diméthyl-guanosine, N²-2-méthyl-guanosine, 7⁺-méthyl-guanosine et 2'-O-méthyl-guanosine.

9. Procédé selon la revendication 8, **caractérisé en ce que** le nucléoside ou l'analogue d'un nucléoside dans le composé (II) ainsi que le nucléophile-nucléoside ou le nucléophile-analogue de nucléoside sont identiques ou différents, de préférence différents.

10. Procédé selon l'une des revendications précédentes, **caractérisé en ce que** ledit procédé est mis en ouvre sous atmosphère de gaz inerte, de préférence sous l'azote gazeux.

11. Composé répondant à la formule générale (IIIa) 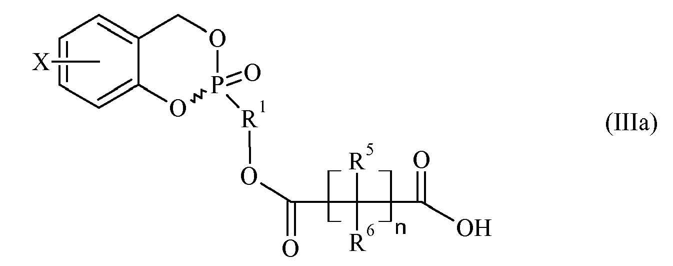 le composé (IIIa) pouvant être pourvu d'un ou de plusieurs substituants X,
X étant un accepteur d'électrons, un H ou un groupe OMe, et Me représentant méthyle,
R¹ étant Nucl, Nucl signifiant nucléoside ou analogue de nucléoside,
n étant égal à 1, 2, 3, 4, 5, 6, 7, 8, 9 ou 10, et
R⁵ et R⁶ représentant, indépendamment l'un de l'autre, H ou des radicaux alkyle ou aryle substitués ou non-substitués, alkyle comprenant des groupes alkyle linéaires et ramifiés, des groupes cycloalkyle, des groupes cycloalkyle pourvus de substituants alkyle, des groupes alkyle pourvus de substituants cycloalkyle ainsi que des groupes alkyle comportant des atomes d'oxygène, d'azote, de soufre ou de phosphore remplaçant un ou plusieurs atomes de carbone du squelette d'hydrocarbure, et aryle désignant des groupes à caractère aromatique y compris les hétéroaryles, les groupes monocycliques aromatiques constitués de 5 à 6 chaînons pouvant contenir zéro à quatre hétéroatomes, et les systèmes polycycliques comportant au moins un cycle aromatique.

12. Compose selon la revendication 11, **caractérisé en ce qu'**il s'agit d'un composé selon la formule générale (IIIb) 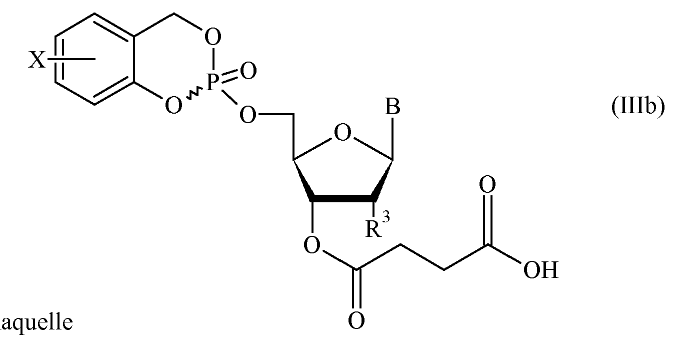 dans laquelle
B est un hétérocycle, de préférence un hétérocycle azoté, s'agissant avec une préférence particulière une des nucléobases guanine, adénine, cytosine, thymine ou uracile, et
R³ représente H, OH ou un groupe protecteur.

13. Composé selon l'une des revendications 11 ou 12, **caractérisé en ce que** X est choisi dans le groupe constitué de H, Me, OMe, MeSO₂, cétone, formyle, ester, C=O, COOH, NO₂ et halogène, Me représentant méthyle, et chaque X pouvant être choisi indépendamment de l'autre si plusieurs substituants de ce type sont présents.

14. Composé selon la revendication 11, **caractérisé en ce que** Nucl est choisi dans le groupe constitué d'adénosine, guanosine, cytidine, thymidine, uridine, désoxyadénosine, désoxyguanosine, inosine, désoxycytidine, désoxyuridine, désoxythymidine, 2-thiocytidine, N⁴-acétyl-cytidine, 2'-O-méthyl-cytidine, 3-méthyl-cytidine, 5-méthyl-cytidine, 2-thiouridine, pseudouridine, dihydrouridine, 5-(carboxyhydroxymethyl)-uridine, 5-carboxymethylaminomethyl-uridine, 5-méthylaminomethyl-uridine, 5-méthoxy-carbonylmethyl-uridine, 5-méthoxy-uridine, ribo-thymidine, 1-méthyl-adénosine, 2-méthyl-adénosine, N⁶-méthyl-adénosine, inosine, 1-méthyl-inosine, guanosine, N²-2,2-diméthyl-guanosine, N²-2-méthyl-guanosine, 7⁺-méthyl-guanosine et 2'-O-méthyl-guanosine.
